(19) Europäisches Patentamt
European Patent Office
Office européen des brevets

(11) **EP 4 130 013 A1**

(12) **EUROPEAN PATENT APPLICATION**
published in accordance with Art. 153(4) EPC

(43) Date of publication:
**08.02.2023 Bulletin 2023/06**

(21) Application number: **21776409.1**

(22) Date of filing: **03.03.2021**

(51) International Patent Classification (IPC):
***C07F 9/09** (1974.07)* ***A61K 9/08** (1974.07)*
***A61K 47/12** (1990.01)* ***A61K 47/24** (1990.01)*
***C07C 53/08** (1968.09)* ***C07C 53/126** (1980.01)*
***C07C 57/12** (1968.09)*

(52) Cooperative Patent Classification (CPC):
**A61K 9/08; A61K 47/12; A61K 47/24; C07C 53/08; C07C 53/126; C07C 57/12; C07F 9/09;** Y02P 20/54

(86) International application number:
**PCT/JP2021/008074**

(87) International publication number:
**WO 2021/192861 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2020 JP 2020056457**

(71) Applicant: **Kyushu University, National University Corporation**
**Nishi-ku**
**Fukuoka-shi**
**Fukuoka 819-0395 (JP)**

(72) Inventor: **GOTO Masahiro**
**Fukuoka-shi, Fukuoka 819-0395 (JP)**

(74) Representative: **Epping - Hermann - Fischer**
**Patentanwaltsgesellschaft mbH**
**Schloßschmidstraße 5**
**80639 München (DE)**

# (54) IONIC LIQUID, SOLVENT, PREPARATION, AND TRANSDERMALLY ABSORBABLE AGENT

(57) An ionic liquid has a structure represented by the following general formula (1). In the general formula (1), R represents a substituted or unsubstituted alkyl group or a substituted or unsubstituted alkenyl group, and at least one ethylene group comprising the alkenyl group may be substituted with a vinylene group. $X^+$ represents a phospholipid with a cationic group.

General formula (1) $R\text{-}COO^- X^+$

FIG. 17

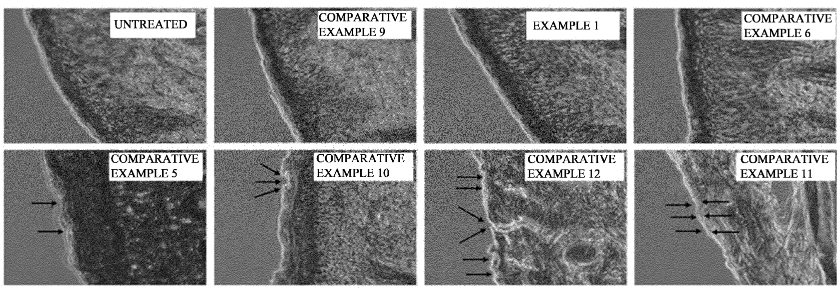


EP 4 130 013 A1

## Description

### Technical Field

The present disclosure relates to an ionic liquid, a solvent, a preparation, and a transdermally absorbable agent.

### Background Art

In recent years, ionic liquids, which are liquids composed only of ions, have been attracting attention. Since ionic liquids, which are salts that exist as liquids over a wide temperature range, have a low melting point and a high solubility, as well as a low volatility and incombustibility, ionic liquids are expected to be applied in a variety of fields such as electrochemical devices, separation and extraction solvents, reaction solvents, and other fields related to tribology and biotechnology. In particular, research is being actively conducted in biotechnology-related fields for the use of ionic liquids as solvents for enzyme reactions, drug delivery, and protein refolding.

As such ionic liquids, Patent Literature 1 proposes an ionic liquid using a phosphonium-type cation (phosphonium-type ionic liquid), and Patent Literature 2 proposes an ionic liquid using an organic amine compound as a cation.

### Citation List

### Patent Literature

Patent Literature 1: Unexamined Japanese Patent Application Publication No. 2020-15688
Patent Literature 2: International Publication No. WO 2009/066457

### Summary of Invention

### Technical Problem

Among the above-described ionic liquids, phosphonium-type ionic liquids are highly irritating, and ionic liquids using organic amine compounds are highly toxic. Therefore, all of these ionic liquids are difficult to be applied to biotechnology-related fields. Therefore, in order to develop ionic liquids with low toxicity, utilization of an amino acid as a cation has also been considered. However, the use of an amino acid as a cation causes an ionic liquid to become hydrophilic, resulting in low solubility in organic solvents and hydrophobic drugs, leading to an inconvenience of greatly restricting applications. As described above, low toxicity is not compatible with solubility in conventional ionic liquids, and conventional ionic liquids cannot be fully utilized in biotechnology-related fields.

The present disclosure was made in view of the above-described circumstances, and an objective of the present disclosure is to provide an ionic liquid that has low toxicity and excellent biocompatibility, and that exhibits high solubility for both a hydrophilic substance and a hydrophobic substance.

### Solution to Problem

As a result of intensive study to solve the above-described problem, the present inventers found that by combining a fatty acid and a phospholipid with a cationic group to form an ionic liquid, an ionic liquid having low toxicity and high solubility in both a hydrophilic substance and a hydrophobic substance can be realized.

An ionic liquid according to a first aspect of the present disclosure has a structure represented by the following general formula (1).

[Chem. 1]

General formula (1) $R\text{-}COO^{-}\ X^{+}$

[In the general formula (1), R represents a substituted or unsubstituted alkyl group or a substituted or unsubstituted alkenyl group, and at least one ethylene group comprising the alkenyl group may be substituted with a vinylene group. $X^{+}$ represents a phospholipid with a cationic group.]

$X^{+}$ in the general formula (1) may be a glycerophospholipid with a cationic group.

$X^{+}$ of the general formula (1) may have a structure represented by the following general formula (2).

[Chem. 2]

General formula (2)

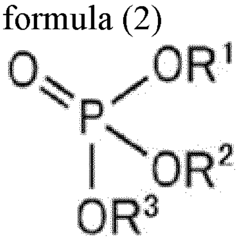

[In the general formula (2), $R^1$ represents an alkyl group substituted with a cationic group, $R^2$ represents a hydrogen atom or a substituted or unsubstituted alkyl group, and $R^3$ represents a substituted or unsubstituted alkyl group.]

$R^3$ in the general formula (2) may be an alkyl group substituted with an alkyl carbonyloxy group.

$R^3$ in the general formula (2) may be an alkyl group substituted with two or more alkyl carbonyloxy groups.

That $R^2$ in the general formula (2) may be a substituted or unsubstituted alkyl group.

The cationic group may be a quaternary ammonium group.

$X^+$ in the general formula (1) may be a derivative of phosphatidylcholine.

The number of carbon atoms of R in the general formula (1) may be from 8 to 22.

That R in the general formula (1) may contain an unsaturated bond.

R in the general formula (1) may have a polyene structure.

R in the general formula (1) may be a group consisting only of carbon atoms and hydrogen atoms.

The ionic liquid of the first aspect of the present disclosure may be hydrophobic.

The ionic liquid of the first aspect of the present disclosure may have a structure represented by the following formula.

[Chem. 3]

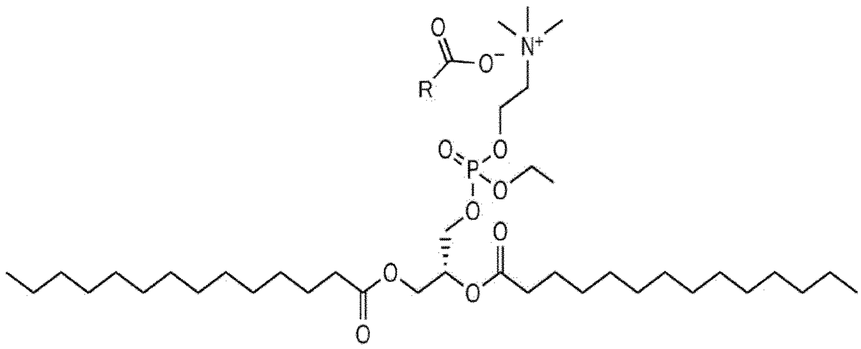

[In the formula, R represents a substituted or unsubstituted alkyl group or a substituted or unsubstituted alkenyl group, and at least one ethylene group comprising the alkenyl group may be substituted with a vinylene group.]

The solvent of a second aspect of the present disclosure includes the above-described ionic liquid of the first aspect of the present disclosure.

The preparation of a third aspect of the present disclosure includes the above-described ionic liquid of the first aspect of the present disclosure.

The transdermally absorbable agent of a fourth aspect of the present disclosure includes the above-described ionic liquid of the first aspect of the present disclosure.

The above-described transdermally absorbable agent of the fourth aspect of the present disclosure may further include a sorbitan fatty acid ester.

The sorbitan fatty acid ester may be a sorbitan monolaurate.

$R\text{-}COO^-$ in the general formula (1) may be a carboxylate ion in which a hydrogen ion may be dissociated from the carboxy group of linoleic acid.

## Advantageous Effects of Invention

The ionic liquid of the present disclosure has low toxicity and excellent biocompatibility, and exhibits high

solubility for both a hydrophilic substance and a hydrophobic substance.

Brief Description of Drawings

FIG. 1 is a diagram illustrating NMR spectra of Ionic Liquids 1 to 3 synthesized in Examples;
FIG. 2 is a diagram illustrating the particle size distribution measured by dynamic light scattering (DLS) for a liquid sample prepared by mixing Ionic Liquid 1 and isopropyl myristate (IPM);
FIG. 3 is a diagram illustrating the particle size distribution measured by DLS for a liquid sample prepared by mixing Ionic Liquid 1 and water;
FIG. 4 is a diagram illustrating the cell viability of epidermal tissues treated with sample solutions containing Ionic liquids 1 to 3 or with various reagents;
FIG. 5A is a diagram illustrating the particle size distribution of Example 1 measured by DLS, FIG. 5B is a diagram illustrating an image of Example 1 imaged by transmission electron microscopy (TEM), and FIG. 5C is a diagram illustrating an image of Example 1 imaged by confocal laser scanning microscopy (CLSM);
FIG. 6A is a diagram illustrating the particle size distribution of Example 2 measured by DLS, FIG. 6B is an image illustrating an image of Example 2 imaged by TEM, and FIG. 6C is a diagram illustrating an image of Example 2 imaged by CLSM;
FIG. 7A is a diagram illustrating the particle size distribution of Example 3 measured by DLS, FIG. 7B is a diagram illustrating an image of Example 3 imaged by TEM, and FIG. 7C is a diagram illustrating an image of Example 3 imaged by CLSM;
FIG. 8 is a diagram illustrating the particle size distribution of droplets of Examples 1 to 3 measured by the CLSM;
FIG. 9A is a diagram illustrating the particle size distribution of Example 1 on Day 90 measured by DLS, FIG. 9B is a diagram illustrating the particle size distribution of Example 2 on Day 90 measured by DLS, and FIG. 9C is a diagram illustrating the particle size distribution of Example 3 on Day 90 measured by DLS;
FIG. 10A is a diagram illustrating the amount of leuprorelin acetate (LA) contained in Example 1 measured by high-pressure liquid chromatography (HPLC), FIG. 10B is a diagram illustrating the amount of LA contained in Example 2 measured by HPLC, and FIG. 10C is a diagram illustrating the amount of LA contained in Example 3 measured by HPLC;
FIG. 11A is a diagram illustrating the encapsulation rate of LA in Examples 1 to 3, and FIG. 11B is a diagram illustrating the maximum loading of LA in samples containing Ionic Liquid 1, 2, or 4;
FIG. 12 is a diagram illustrating the change over time of the concentration of LA in the receiver phase in a skin penetration test;
FIG. 13 is a diagram illustrating the amount of LA in dermal and topical LA after 36 hours in a skin penetration test;
FIG. 14 is a diagram illustrating the concentration of LA in plasma in an *in vivo* pharmacokinetic study;
FIG. 15 is a diagram illustrating the cell viability of epidermal tissue treated with a formulation containing Ionic Liquid 1;
FIG. 16 is a diagram illustrating the change overtime in body weight of mice treated with a formulation containing Ionic Liquid 1 by transdermal administration; and
FIG. 17 is a diagram illustrating an image of the stratum corneum of mice treated with a formulation containing Ionic Liquid 1 by dermal administration.

Description of Embodiments

Embodiments of the present disclosure are described in detail below. Description of constituent requirements described below may be based on representative embodiments and specific examples, but the present disclosure is not limited to such embodiments. As used herein, a numerical value range expressed using "from A to B" means a range that includes the numerical values A and B as the lower and upper limits. The isotopic species of hydrogen atoms present in molecules of a compound used in the present disclosure is not particularly limited. For example, all hydrogen atoms in a molecule may be $^1H$, or some or all may be $^2H$ (deuterium D).

<Ionic Liquid>

The ionic liquid according to the present embodiment has a structure represented by the following general formula (1).
[Chem. 4]

General formula (1) $R\text{-}COO^- X^+$

In the general formula (1), R represents a substituted or unsubstituted alkyl group or a substituted or unsubstituted alkenyl group, and at least one ethylene group comprising an alkenyl group may be substituted with a vinylene group.

An alkyl group in R may be linear, branched, or cyclic. The number of carbon atoms of an alkyl group in R is preferably from 8 to 22, and more preferably from 12 to 22. Examples of an alkyl group in R include a linear alkyl group such as a dodecyl group, a tridecyl group, a tetradecyl group, a pentadecyl group, a hexadecyl group, a heptadecyl group, an octadecyl group, a nonadecyl group, an icosyl group, a henicosyl group, or a docosyl group, a branched alkyl group thereof, and a cyclic alkyl group thereof. Examples of a substituent that can be substituted on an alkyl group include an amino group, a benzyl group, and a halogen atom (for example, a fluorine atom). These substituents may be further substituted with a substituent. When an alkyl group is substituted with a substituent, the total of the number of carbon atoms of the alkyl group and the number of carbon atoms of the substituent is preferably from 8 to 22, and more preferably from 12 to 22.

An alkenyl group in R may be linear or branched. The number of carbon atoms of an alkenyl group in R is preferably from 8 to 22, and more preferably from 12 to 22. Examples of an alkenyl group in R include a linear alkenyl group such as a dodecenyl group, a tridecenyl group, a tetradecenyl group, a pentadecenyl group, a hexadecenyl group, a heptadecenyl group, an octadecenyl group, a nonadecenyl group, an icosenyl group, a henicosenyl group, or a docosenyl group, and a branched alkenyl group thereof. Examples of a substituent that can be substituted on an alkenyl group include an amino group, a benzyl group, and a halogen atom (for example, a fluorine atom). These substituents may be further substituted with a substituent. When an alkenyl group is substituted with a substituent, the total of the number of carbon atoms of the alkenyl group and the number of carbon atoms of the substituent is preferably from 8 to 22, and more preferably from 12 to 22. An alkenyl group in R may have at least one ethylene group substituted with a vinylene group to form a polyene structure. The number of double bonds in the polyene structure is preferably from 2 to 6, and more preferably from 2 to 4. The position of double bonds is not particularly limited, and it is preferable that the double bonds are arranged with at least two single bonds separating them from each other.

Among these, R is preferably a group with an unsaturated bond, and is more preferably a substituted or unsubstituted alkenyl group or a substituted or unsubstituted polyene structure. An alkyl group, an alkenyl group, or a group having a polyene structure in R may be substituted with a substituent, and even in such a case, R is preferably consisting only of carbon atoms and hydrogen atoms. Specifically, when an alkyl group, an alkenyl group, and a polyene structure is substituted with a substituent, the substituent is also preferably consisting only of carbon atoms and hydrogen atoms.

As $R\text{-}COO^-$, for example, a carboxylate ion or a derivative thereof, in which a hydrogen ion is dissociated from a carboxy group of a fatty acid, can be used. A fatty acid that generates a carboxylate ion may be a saturated fatty acid or an unsaturated fatty acid. Examples of a saturated fatty acid include myristic acid (C14:0), palmitic acid (C16:0), stearic acid (C18:0), and lauric acid (C12:0), and examples of an unsaturated fatty acid include oleic acid (C18:1), linoleic acid (C18:2), α-linolenic acid (C18:3), γ-linolenic acid (C18:3), arachidonic acid (C20:4), icosapentaenoic acid (C20:5), docosahexaenoic acid (C22:6), and erucic acid (C22:1). Here, numbers in parentheses are the number of carbon atoms and the number of double bonds in each fatty acid. For example, (C18:2) for linoleic acid indicates that the number of carbon atoms is 18 and the number of double bonds is two.

In general formula (1), $X^+$ represents a phospholipid with a cationic group. Here, "phospholipid" means a lipid including a phosphate ester structure, and "cationic group" means a positively charged substituent. $X^+$ is preferably a glycerophospholipid with a cationic group, and is more preferably a derivative of phosphatidylcholine. A derivative of phosphatidylcholine is a compound including a glycerol backbone and substituted or unsubstituted alkanoyl groups attached to the 1- and 2-positions of the glycerol backbone, respectively, a phosphate group (-P(O)(OH)O-) bound to the 3-position of the glycerol backbone, and a choline residue attached to this phosphate group. In this phosphatidylcholine derivative, at least one ethylene group of an alkanoyl group may be substituted with a vinylene group, and a hydrogen atom of a hydroxyl group of a phosphate group may be substituted with a substituted or unsubstituted alkyl group.

$X^+$ is preferably a phospholipid having a structure represented by the following general formula (2), and is more preferably a glycerophospholipid having a structure represented by the following general formula (2).

[Chem. 5]

General formula (2)

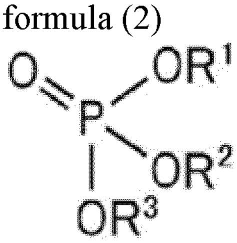

In the general formula (2), $R^1$ represents an alkyl group substituted with a cationic group, $R^2$ represents a hydrogen atom or a substituted or unsubstituted alkyl group, and $R^3$ represents a substituted or unsubstituted alkyl group.

An alkyl group in $R^1$ may be linear, branched or cyclic. The number of carbon atoms of an alkyl group in $R^1$ is preferably from 1 to 20, more preferably from 1 to 10, and further preferably from 1 to 6. Examples of an alkyl group in $R^1$ include a methyl group, an ethyl group, an n-propyl group, and an isopropyl group, and an alkyl group in $R^1$ is preferably an ethyl group.

A cationic group in $R^1$ is preferably an ammonium group represented by the following general formula (3).

[Chem. 6]

General formula (3) $* - NR^4{}_3{}^+$

In the general formula (3), $R^4$ represents a hydrogen atom or a substituted or unsubstituted alkyl group, and * represents a bonding position to an alkyl group. Three $R^4$s may be identical or different from each other. The number of the three $R^4$s that are substituted or unsubstituted alkyl groups is not particularly limited, and all of the $R^4$s may be hydrogen atoms or substituted or unsubstituted alkyl groups, or one or two of them may be hydrogen atoms and the remainder may be substituted or unsubstituted alkyl groups.

An alkyl group in $R^4$ may be linear, branched or cyclic. The number of carbon atoms of an alkyl group in $R^4$ is preferably from 1 to 20, more preferably from 1 to 10, and more preferably from 1 to 6. Examples of an alkyl group in $R^4$ include a methyl group, an ethyl group, an n-propyl group, and an isopropyl group, and an alkyl group in $R^4$ is preferably a methyl group. Examples of a substituent that can be substituted on an alkyl group include a benzyl group and a halogen atom (for example, a fluorine atom). These substituents may be further substituted with a substituent.

A cationic group in $R^1$ is preferably a quaternary ammonium group, and is more preferably a quaternary ammonium group represented by the general formula (5) (an ammonium group in which all of $R^4$s are substituted or unsubstituted alkyl groups). The position of substitution of a cationic group in an alkyl group is not particularly restricted, and it is preferable that a hydrogen atom bonded to a carbon atom at the end of an alkyl group is substituted with a cationic group.

In the general formula (2), $R^2$ represents a hydrogen atom or a substituted or unsubstituted alkyl group. $R^2$ is preferably a substituted or unsubstituted alkyl group. The alkyl group in $R^2$ may be linear, branched or cyclic. The number of carbon atoms of an alkyl group in $R^2$ is preferably from 1 to 20, more preferably from 1 to 10, and more preferably from 1 to 6. Examples of an alkyl group in $R^2$ include a methyl group, an ethyl group, an n-propyl group, and an isopropyl group, and it is preferable that the alkyl group in $R^2$ is an ethyl group. Examples of a substituent that can be substituted on an alkyl group include a benzyl group and a halogen atom (for example, a fluorine atom). These substituents may be further substituted with a substituent.

In the general formula (2), $R^3$ represents a substituted or unsubstituted alkyl group. An alkyl group in $R^3$ may be linear, branched or cyclic. The number of carbon atoms of an alkyl group in $R^3$ is preferably from 1 to 20, more preferably from 1 to 10, and further preferably from 1 to 6. Examples of an alkyl group in $R^3$ include a methyl group, an ethyl group, an n-propyl group, and an isopropyl group, and the alkyl group in $R^3$ is preferably an n-propyl group. Examples of a substituent that can be substituted on an alkyl group include an alkyl carbonyloxy group, a benzyl group, and a halogen atom (for example, a fluorine atom). These substituents may be further substituted with a substituent.

Preferable examples of $R^3$ include an alkyl group substituted with an alkyl carbonyloxy group, and a more preferable $R^3$ is an alkyl group substituted with two or more alkyl carbonyloxy groups. Here, the two or more alkyl carbonyloxy groups may be the same or different from each other, and are preferably the same.

The number of substituents of alkyl carbonyloxy groups in an alkyl group is preferably from 2 to 8, more preferably from 2 to 4, and most preferably 2. The position of substitution of an alkyl carbonyloxy group in an alkyl group is not particularly restricted, and when an alkyl group as the main chain of $R^3$ is an n-propyl group, a molecule in which a hydrogen atom bound to the terminal carbon atom and a hydrogen atom bound to the adjacent carbon atom are substituted with an alkyl carbonyloxy group corresponds to a glycerophospholipid, which is a particularly preferable phospholipid.

For a description, a preferable range, and specific examples of an alkyl group in an alkyl carbonyloxy group, the description, preferable range, and specific examples of an alkyl group in R above can be referred to.

In the following, examples of a preferable compound of an ionic liquid having a structure represented by the general formula (1) are provided. However, the ionic liquid according to the present embodiment should not be construed as being limited by these specific examples. In the following formula, $R-COO^-$ is synonymous with $R-COO^-$ in the general formula (1), and specific examples thereof include a respective carboxylate ion of linoleic acid, oleic acid, acetic acid, or stearic acid.

[Chem. 7]

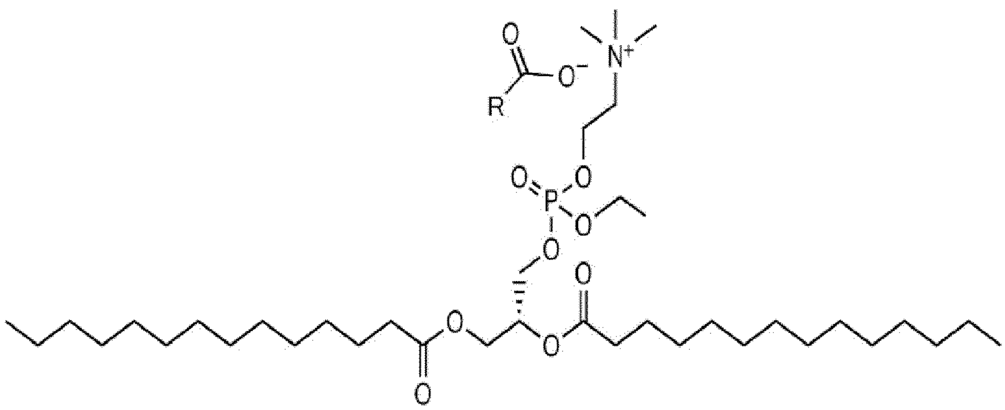

[Property of Ionic Liquid]

The following is a description of preferable properties of an ionic liquid.

(Hydrophobicity)

The ionic liquid of the present embodiment is preferably hydrophobic. Here, "hydrophobic" means that the liquid dissolves in IPM by 0.1% by weight or more.

The ionic liquid according to the present embodiment preferably dissolves in IPM by 0.1% by weight or more (hydrophobic), more preferably by 5% by weight or more, and further preferably by 20% by weight or more. Here, dissolution of an ionic liquid in IPM encompasses not only dissolution of an ionic liquid in IPM forming a homogeneous system, but also dissolution of an ionic liquid in IPM forming an inverse micelle. Formation of an inverse micelle by an ionic liquid in IPM can be confirmed by the presence of a peak in the particle size distribution measured by DLS.

The ionic liquid of the present embodiment has a structure represented by the general formula (1), and can be easily made hydrophobic since R and $X^+$ contain an alkyl group or an alkenyl group. An ionic liquid that is hydrophobic exhibits high compatibility with a hydrophobic solvent, an oily base material, a hydrophobic drug, and the like, and can therefore be easily mixed with them. An ionic liquid has high permeability to a permeation barrier of a stratum corneum, and can be easily absorbed transdermally. Therefore, an ionic liquid, which is hydrophobic, can be utilized especially for transdermal drug delivery systems (DDS).

(Micelle or Liposome Forming Ability)

The ionic liquid of the present embodiment is preferably hydrophobic and, when mixed with water at a concentration of up to 20%, for example, and a particle size distribution of the liquid mixture is measured by DLS, a peak preferably appears, and the peak preferably appears in the range of from 0.01 to 1 μm. A peak appearing in a particle size distribution indicates that an ionic liquid is dissolved in the liquid mixture, forming micelles or liposomes with the particle size of the peak. Since such an ionic liquid dissolves in a hydrophobic solvent with high compatibility and form a micelle or liposome in a hydrophilic solvent to uniformly disperse (micellar dissolution), favorable solubility can be obtained in both a hydrophobic solvent and a hydrophilic solvent. Such a micelle or liposome can be utilized for a variety of applications. For example, an ionic liquid that forms a micelle or liposome can be used as a carrier for a drug delivery system by encapsulating a variety of molecules inside the micelle or liposome.

(Melting Point)

The melting point of the ionic liquid according to the present embodiment is preferably 100°C or less, more preferably 60°C or less, and further preferably 40°C or less. Here, the melting point is defined as the melting point as determined by differential scanning calorimetry. An ionic liquid with a melting point in the above-described range can be suitably used as a solvent since the liquid exhibits a liquid state over a wide range of temperatures.

<Solvent>

The solvent according to the present embodiment encompasses the ionic liquid according to the present embodiment. An ionic liquid contained in the solvent according to the present embodiment may consist of only one type of compounds represented by the general formula (1), or may contain two or more types of the compounds. The solvent according to the present embodiment may consist of only the ionic liquid according to the present embodiment, or may contain another solvent. The other solvent is not particularly restricted, and may be selected from known solvents as appropriate.

The ionic liquid according to the present embodiment has a structure represented by the general formula (1), and has high compatibility with a hydrophobic substance since R and $X^+$ contain an alkyl or an alkenyl group, and exhibits surfactant-like behavior toward a hydrophilic substance due to the presence of a carboxylate ion and a cationic group. Therefore, the ionic liquid according to the present embodiment can be homogeneously mixed in combination with a hydrophobic or a hydrophilic solvent, and can dissolve both a hydrophobic solute and a hydrophilic solute. For example, many pharmacologically active substances are generally difficult to dissolve, but when the ionic liquid of the present embodiment is used as a solvent, such a poorly soluble pharmacologically active substance can also be dissolved. By using such an ionic liquid, a solvent that exhibits high solubility can be realized regardless of a solvent and a solute to be combined with the ionic liquid. A preparation and transdermally absorbable agent with excellent biocompatibility can be realized.

<Preparation>

The preparation according to the present embodiment encompasses the ionic liquid according to the present embodiment. An ionic liquid contained in the preparation according to the present embodiment may be one of the compounds represented by the general formula (1), or may be two or more of the compounds represented by the general formula (1). In addition to the ionic liquid of the present embodiment, the preparation according to the present embodiment may contain a component normally used in preparations, such as an active ingredient, an additive, an excipient, or a base agent. The form of the preparation according to the present embodiment is not particularly restricted and may be in any form, for example, oral, topical, and injectable.

The ionic liquid according to the present embodiment is a combination of an anion with a fatty acid basic backbone and a cation with a phospholipid basic backbone, and both basic backbones are biologically relevant substances, resulting in low toxicity and high biocompatibility. The ionic liquid of the present embodiment is highly compatible with a hydrophobic substance since R and $X^+$ contain an alkyl or an alkenyl group, and the ionic liquid exhibits surfactant-like behavior toward a hydrophilic substance due to the presence of a carboxylate ion and a cationic group. Therefore, by using the ionic liquid according to the present embodiment, a preparation that has advantages of an ionic liquid and is safe can be easily prepared.

<Transdermally Absorbable Agent>

The transdermally absorbable agent according to the present embodiment contains the ionic liquid according to the present embodiment. An ionic liquid contained in the transdermally absorbable agent according to the present embodiment may be one, two or more of the compounds represented by the general formula (1).

The ionic liquid according to the present embodiment has low toxicity, high biocompatibility, and can permeate a stratum corneum of a skin since an alkyl group or an alkenyl group and a lipid structure are contained in the molecule, thereby exhibiting hydrophobic properties. Therefore, the ionic liquid according to the present embodiment has advantages of an ionic liquid, is safe, and can realize a transdermally absorbable agent that exhibits favorable transdermal absorption properties.

The formulation of the transdermally absorbable agent is not particularly restricted and can be, for example, a liquid (for example, a lotion formulation or a spray formulation), an ointment, a cream, a gel, an emulsion, and a patch formulation. In addition to the ionic liquid according to the present embodiment, the transdermally absorbable agent may contain an active ingredient and a base agent. A base material can be selected from among those normally used for transdermally absorbable agents. An additive agent such as a stabilizer, a preservative, a dissolution aid, an emulsifier,

a suspending agent, a pH adjuster, or an antioxidant used in pharmaceuticals, quasi-drugs, and cosmetics such as topical agents may be added to a transdermally absorbable agent, if necessary.

In addition to an ionic liquid that functions like a surfactant, a transdermally absorbable agent may further contain an auxiliary surfactant. Examples of the auxiliary surfactant include a sorbitan fatty acid ester. Examples of the sorbitan fatty acid ester include sorbitan monolaurate, sorbitan monostearate, sorbitan tristearate, sorbitan monooleate, sorbitan trioleate, sorbitan sesquioleate, and sorbitan monopalmitate. The sorbitan fatty acid ester is suitably sorbitan monolaurate (span-20).

The content of an auxiliary surfactant in a transdermally absorbable agent is set appropriately, and is, for example, from 1 to 10% by mass volume (w/v), from 2 to 8 w/v%, from 3 to 7 w/v%, and from 4 to 6 w/v%, and preferably 5 w/v%. The content of an ionic liquid in a transdermally absorbable agent is set appropriately, and is, for example, from 1 to 10% by mass volume (w/v), from 2 to 8 w/v%, from 3 to 7 w/v%, and from 4 to 6 w/v%, and preferably 5 w/v%. The mass ratio of the above-described ionic liquid to the auxiliary surfactant in a transdermally absorbable agent may be, for example, 1:0.6, 1:0.7, 1:0.8, 1:0.9, 0.6:1, 0.7:1, 0.8:1 or 0.9:1, and is preferably 1:1.

When an ionic liquid is contained in a transdermally absorbable agent, the ionic liquid is preferably a carboxylate ion in which R-COO$^-$ in the above-described general formula (1) is a carboxylate ion in which a hydrogen ion is dissociated from a carboxylic group of linoleic acid.

## Examples

The following Examples and Comparative Examples are provided to further describe features of the present disclosure in detail. The materials, amounts used, percentages, treatment details, treatment procedures, and the like described in the following Examples may be changed as appropriate without departing from the spirit of the present disclosure. Therefore, the scope of the present disclosure should not be construed as being limited by the following specific examples. Melting points were measured using a simultaneous differential thermogravimetric analyzer (Hitachi High-Technologies Corporation, TG/DTA 7300), and DLS measurements were performed using a Zetasizer (Malvern Panalytical Corporation, Malvern-UK. Nano series).

### [1] Synthesis of Ionic Liquid

### (Synthesis Example 1) Synthesis of Ionic Liquid 1

[Chem. 8]

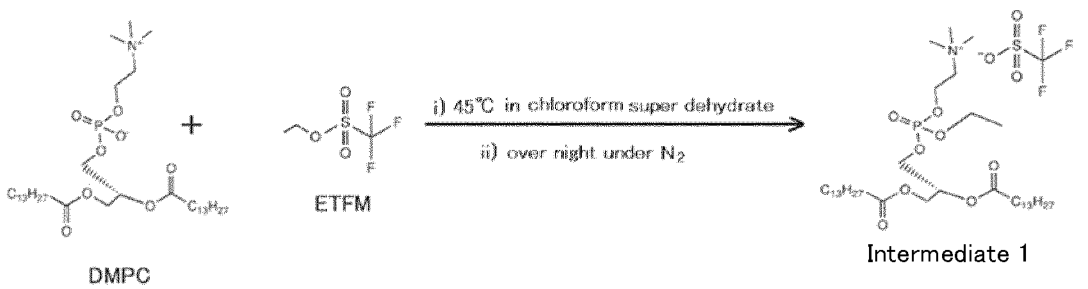


DMPC ETFM Intermediate 1

1,2-Dimyristoyl-sn-glycero-3-phosphatidylcholine (DMPC), ethyl trifluoromethanesulfonate (ETFM) (1 molar equivalent), and chloroform (superhydrated) were placed in a vessel and stirred overnight at 45°C under a nitrogen atmosphere. This reaction solution was dried overnight under a light-shielded nitrogen atmosphere to yield Intermediate 1 (trifluoromethanesulfonate salt of 1,2-dimyristoyl-sn-glycero-3-ethylphosphatidylcholine (EDMPC)).

[Chem. 9]

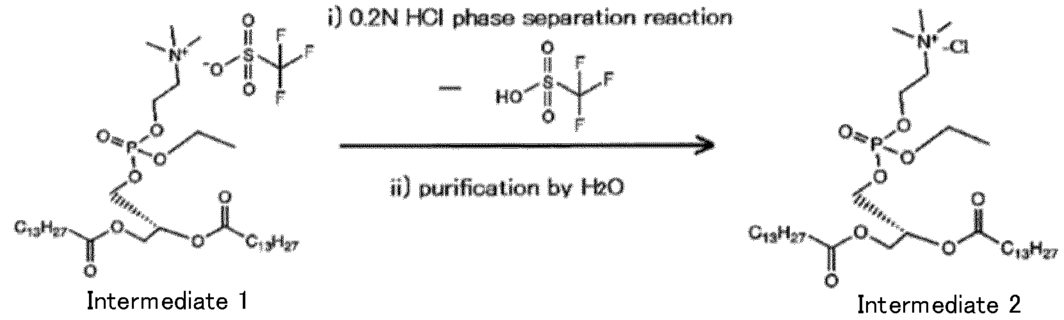


After dissolving Intermediate 1 in chloroform, 0.2 N hydrochloric acid is added and a phase separation reaction is performed to obtain a lower layer containing Intermediate 2 (EDMPC chloride) and an upper layer (aqueous phase) containing trifluoromethanesulfonic acid. The upper layer was removed, the lower layer was washed with milli-Q water to remove unreacted hydrochloric acid, and chloroform and excess water were removed by an evaporator and a refrigerated dryer to obtain Intermediate 2.

[Chem. 10]

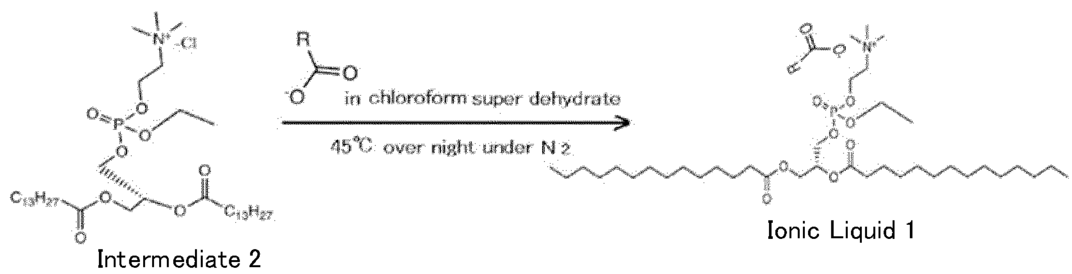


Linoleic acid (1 molar equivalent) and chloroform (superhydrated) were added to Intermediate 2, and reacted under a light-shielded dry nitrogen atmosphere at 45°C overnight with stirring to obtain a desired Ionic Liquid 1. The NMR spectrum of the obtained Ionic Liquid 1 is illustrated in FIG. 1.

(Synthesis Examples 2 and 3) Synthesis of Ionic Liquid 2, 3 and 4

Ionic Liquid 2 containing a carboxylate ion of oleic acid, Ionic Liquid 3 containing a carboxylate ion of acetic acid, and Ionic Liquid 4 containing a carboxylate ion of stearic acid were synthesized in the same manner as in Synthesis Example 1, except that oleic acid, acetic acid or stearic acid was used in place of linoleic acid. The NMR spectra of the obtained Ionic Liquids 2 and 3 are illustrated in FIG. 1.

[2] Evaluation

The synthesized Ionic Liquids 1 to 3 were evaluated for melting point measurement, DLS measurement, solubility, and toxicity. In the following, Ionic Liquid 1 is sometimes referred to as [EDMPC][Lin], Ionic Liquid 2 as [EDMPC][Ole], Ionic Liquid 3 as [EDMPC][Act], and Ionic Liquid 4 as [EDMPC][Ste].

(Measurement of Melting Point)

The melting points were measured by differential scanning calorimetry: 14.8°C for Ionic Liquid 1, 34.5°C for Ionic Liquid 2, and 47.5°C for Ionic Liquid 3, all indicating low melting points below 50°C.

(DLS Measurement)

Two liquid samples of each ionic liquid were prepared by mixing each ionic liquid with IPM or water at a concentration of 5% by weight, and the particle size distribution of each was measured by DLS. As representative, the

particle size distribution of Ionic Liquid 1 in IPM is illustrated in FIG. 2, and the particle size distribution in water is illustrated in FIG. 3. As illustrated in FIGS. 2 and 3, peaks were observed at specific particle diameters in both liquid samples with IPM as a solvent and in liquid samples with water as a solvent. Similar peaked particle size distributions were measured for the other ionic liquids in liquid samples. The observed peak in the particle size distribution in water indicates that Ionic Liquids 1 to 3 were hydrophobic and formed micelles and dispersed uniformly (micellar dissolution) in water by exhibiting surfactant-like behavior. Peaks were also observed in the particle size distribution of IPM, suggesting that Ionic Liquids 1 to 3 may form inverse micelles in hydrophobic solvents.

(Evaluation of Solubility)

The solubility of ionic liquids in a variety of solvents was evaluated at room temperature. Specifically, Ionic Liquids 1 to 3 were placed in a glass tube with respective solvents and mixed by stirring with a vortex mixer for from 1 to 2 minutes. The ratio of Ionic Liquids 1 to 3 to the corresponding solvents was 50:50 by weight. The solubility and transparency of the obtained liquids (liquid samples) were visually observed and evaluated according to the following criteria. The results are listed in Table 1.

◦: Ionic liquid is dissolved uniformly and the clarity of the liquid sample is high
Δ: Ionic liquid is uniformly dissolved, but liquid sample is cloudy
× : Ionic liquid and solvent are separated, and the ionic liquid is not dissolved in the solvent.

The same evaluation was performed for intermediate 2 ([EDMPC][Cl]) as a comparison.

[Table 1]

| Solvent | Ionic Liquid 1 [EDMPC] [Lino] | Ionic Liquid 2 [EDMPC] [Olc] | Ionic Liquid 3 [EDMPC] [Act] | Intermediate 2 [EDMPC] [Cl] |
|---|---|---|---|---|
| Isopropyl myristate | ○ | ○ | ○ | ○ |
| Isopropyl alcohol | ○ | ○ | ○ | ○ |
| Ethanol | ○ | ○ | ○ | ○ |
| Dimethyl sulfoxide | ○ | ○ | ○ | ○ |
| Water | Δ | Δ | Δ | × |
| Phosphate-buffered saline | Δ | Δ | Δ | × |
| n-Hexane | ○ | ○ | ○ | × |
| Cyclohexane | ○ | ○ | ○ | × |
| Heptane | ○ | ○ | ○ | × |
| Toluene | ○ | ○ | ○ | × |

As illustrated in Table 1, Intermediate 2 with chlorine ion as an anion dissolved in IPM and isopropyl alcohol, but not in water-containing solvents (water, phosphate-buffered saline) or nonpolar solvents (n-hexane, cyclohexane, heptane, toluene). On the other hand, Ionic Liquids 1 to 3, which used fatty acid carboxylate ions as anions, all exhibited high solubility in the various solvents. The cloudiness of liquid samples using water and phosphate-buffered saline as solvents was considered to be because each ionic liquid dissolved in these solvents by forming micelles. Combination of a phospholipid with a cationic group with a fatty acid carboxylate ion also improves solubility in aqueous and nonpolar solvents, resulting in high solubility in both nonpolar and polar solvents, hydrophobic and hydrophilic solvents.

(Cytotoxicity Test)

A cytotoxicity test was performed on human artificial epidermis (manufactured by Japan Tissue Engineering Co. Ltd.: LabCyte EPI-MODEL 12 cells) using the MTT cell viability assay. The MTT cell viability assay measures cell viability by utilizing conversion of yellow MTT (3-(4,5-dimethyl-thiazol-2-yl)-2,5-diphenyltetrazolium bromide) to blue

formazan by dehydrogenase in intracellular mitochondria. The higher the amount of formazan produced (higher absorbance at 570 nm), the higher the cell viability.

Specifically, sample solutions were first prepared by dissolving Ionic Liquids 1 to 3 in IPM. Here, the concentration of ionic liquid in each sample solution was 5% by weight, 10% by weight, 20% by weight, 50% by weight, or 100% by weight.

On the other hand, a 24-well plate injected with assay medium (500 μL) was prepared, and a culture cup of human epidermal cells was attached in each well. The 24-well plate was incubated at 37°C for 24 hours under a humidified atmosphere of 5% $CO_2$, and then a sample solution (25 μL) and a phosphate-buffered saline (PBS: 25 μL) as a control were injected into each culture cup and incubated at 37°C for 24 hours under a humidified atmosphere of 5% $CO_2$. After incubation, the sample solution was removed from each culture cup and the tissue surface in the cup was washed 15 times with Dulbecco's phosphate-buffered saline. Then, 0.5 mg/mL ofMTT assay medium (500 μL) was injected into each culture cup and incubated at 37°C for 3 hours under a 5% $CO_2$ atmosphere. After incubation, epidermal tissue was removed from the culture cup and transferred into a microtube containing 2-propanol (300 μL) and left under dark conditions at room temperature for 48 hours to extract a formazan that had developed in the epidermal tissue into 2-propanol. This tissue extract (100 μL) and 2-propanol (blank) were injected into each well of a 96-well ELISA plate, and absorbance was measured at 650 nm and 570 nm, respectively, and cell viability was calculated by the following formula.

$$\text{Cell viability (\%)} = [\text{A (Sample solution)} / \text{A(PBS)}] \times 100$$

In the formula, A (sample solution) represents the absorbance at 570 nm of the tissue extract treated with each sample solution, and A (PBS) represents the absorbance at 570 nm of the tissue extract treated with phosphate-buffered saline.

Three tests were performed as described above, and results of the mean value and the standard deviation (SD) of the cell viability are illustrated in FIG. 4. In FIG. 4, the abbreviations and ionic liquids listed along the horizontal axis indicate the type of a reagent used for treatment of epidermal tissue or an ionic liquid included in a sample solution. [Choline][Ole], emim Tf2SA, and SDS are Comparative Examples, respectively, and [Choline][Ole] represents an ionic liquid of choline and oleic acid, emim Tf2SA represents a commercially available ionic liquid (1-ethyl-3-methylimidazolium bis(trifluoroethylsulfonyl)amide), and SDS represents sodium lauryl sulfate (an anionic surfactant).

As illustrated in FIG. 4, high cell viability was obtained for ionic liquids 1 to 3 even when used at a concentration of 20% by weight, suggesting that there was no toxicity problem. For Ionic Liquids 1 and 2, cell viability was also measured at 100% concentration, and also in this case, much higher cell viability than 20% SDS was obtained. From these results, it was found that an ionic liquid, a combination of a phospholipid with a cationic group and a carboxylate ion of a fatty acid, is extremely low in toxicity.

Application of ionic liquids 1, 2, and 4 to transdermally absorbable agents for administration of LA through a skin was examined as follows. Statistical analysis in the following was performed by two-way analysis of variance and Tukey's test based on Dagnett's multiple comparison method and prism6 (manufactured by GraphPad Software, Inc.). Statistical significance was set at *p<0.05, **p<0.01, ***p<0.001, ****p<0.0001 and *****p<0.00001.

(Preparation of Sample)

2 mL of 3.0 mg/mL aqueous LA solution and 4 mL of 12.5 mg/mL cyclohexane solution of Ionic Liquid 1, Ionic Liquid 2, or Ionic Liquid 4 were homogenized at 26,000 rpm for 2 minutes using a homogenizer (manufactured by POLYTRON, high-speed homogenizer, PT2500E). The resulting solution was lyophilized to remove water and cyclohexane to obtain an ionic liquid-peptide complex in powder form. The ionic liquid-peptide complex was stirred in 1 mL of IPM solution containing 5% span-20 for 12 hours to obtain an ionic liquid/oil-nanodispersion system (IL/O-ND).

(Characterization of II,/O-ND)

Samples 1, 2 or 3 were evaluated by DLS for particle size, polydispersity index (PDI), and peak intensity. The average of 10 measurements for each sample was taken as the particle size. Particle size and shape were analyzed by TEM. In TEM, a 2 μL sample was placed on a carbon-copper film TEM grid and incubated for 2 minutes to allow the film to absorb the sample. The IPM was washed with cyclohexane and incubated for another 2 minutes. The deposited complexes were stained with 2% uranyl acetate solution. Images of the samples were then observed using TEM-2010 (JEOL) at 120 kV. Morphological analysis of the sample droplet diameter and shape was performed by CLSM using an LSM700 (manufactured by Carl Zeiss) in bright field. The CLSM captured images at 63oil×3 resolution.

FIGS. 5A, 6A, and 7A illustrate DLS results for Examples 1, 2, and 3, respectively. The particle sizes of the droplets in Examples 1, 2 and 3 were 251 nm, 220 nm and 265 nm, respectively. FIGS. 5B, 6B, and 7B illustrate images

of Examples 1, 2, and 3 imaged by TEM, respectively. In the TEM images, slightly deformed particles of Examples 1 to 3 were observed. FIGS. 5C, 6C, and 7C illustrate images of Examples 1, 2, and 3 imaged by CLSM, respectively. In the CLSM images, Examples 1 to 3 formed uniform particles, and the particles diffused freely by forming nanoparticles. Particle size, PDI, and peak intensity measured by DLS are listed in Table 2. FIG. 8 illustrates the droplet diameters of Examples 1 to 3 as measured by CLSM. Particle sizes measured by CLSM were distributed from 200 to 300 nm, supporting the DLS results.

[Table 2]

| | Leuprorelin acetate 3 mg/mL | | | Particle size (nm) | PDI | Peak intensity (%) |
|---|---|---|---|---|---|---|
| | Surfactant | Auxiliary surfactart | Basic liquid | | | |
| Example 1 | 5% [EDMPC][Lin] | 5% span-20 | 1mL IPM | 251 | 0.26 | 99% |
| Example 2 | 5% [EDMPC][Ole] | 5% span-20 | 1mL IPM | 220 | 0.44 | 100% |
| Example 3 | 5% [EDMPC][Ste] | 5% span-20 | 1mL IPM | 265 | 0.39 | 98% |

(Evaluation of Stability of II,/O-ND)

The physicochemical stability of LA in IL/O-ND was evaluated by measuring particle size and quantifying LA by DLS and HPLC over 90 days for samples stored at low temperature (-4°C), room temperature (25°C), and high temperature (37°C). For particle size, droplet size was measured by DLS as described above.

For evaluation by HPLC, a linear correlation curve of LA obtained using dilutions (25% acetonitrile solution containing 1% TFA) with concentrations ranging from 0.01 to 0.1 μg/mL was considered. Test samples were prepared by centrifuging the diluent with a concentration of 0.1 μg/mL of LA at 10,000 rpm for 30 minutes. An HPLC system (manufactured by Japan Spectroscopy Corporation) was used for HPLC measurements. A solution containing 0.0087 M ammonium monophosphate and acetonitrile in a volume ratio of 77:23, adjusted to pH 6.5 with HaOH/HCl, was used as a mobile phase. Chromatographic separation was performed at 30°C using an Inert sustain ODS column (150 × 4.6 mm, 5 μm, manufactured by G.L. Science Inc.). The injected 100 μL test sample was separated at a flow rate of 1.0 mL/min of mobile phase at 220 nm.

According to the DLS results, the particle size of Examples 1, 2, and 3 remained constant (from 200 to 300 nm) at room temperature at least up to 90 days, but tended to decrease by from 50 to 60 nm at higher temperatures and increase by from 700 to 1,000 nm at lower temperatures. FIGS. 9A, 9B and 9C illustrate the particle size at 90 days for Examples 1, 2 and 3, respectively.

FIGS. 10A, 10B and 10C illustrate the amount of LA contained in Examples 1, 2 and 3, respectively, as measured by HPLC. The amount of LA tended to decrease slightly at lower temperatures, but not significantly, and remained stable with little change at any temperature.

(Evaluation of LA Encapsulation Amount into II,/O-ND)

The II,/O-ND solution encapsulating LA was centrifuged at 10,000 rpm for 30 minutes to obtain a supernatant. The amount of LA encapsulated in IL/O-ND was evaluated by measuring the concentration of unencapsulated LA leaked into the supernatant by HPLC. The concentration of unencapsulated LA leaked into the supernatant was determined based on a calibration curve prepared from a standard solution of LA, and the encapsulation rate of LA in the IL/O-ND was calculated. IPM containing 3 mg/mL LA and 5% span-20 was used as Comparative Example 1 and IPM containing 3 mg/mL LA and 5% ionic liquid 1 as Comparative Example 2.

(Evaluation of Maximum LA Capacity in IL/O-ND)

In order to evaluate the maximum LA loading capacity in IL/O-ND, an excess amount of LA was added to IPM containing 5% Ionic Liquid 1, 2, or 4 and 5% span-20 and continuously stirred. LA not loaded on IL/O-ND was removed using a centrifugation process. The concentration of LA in samples was determined by HPLC. IPM containing 5% span-20 was used as Comparative Example 3 and IPM containing 5% Ionic Liquid 1 as Comparative Example 4.

FIG. 11A illustrates the LA encapsulation rates in Examples 1 to 3. Addition of Ionic Liquid 1, 2, or 4 and auxiliary surfactants to IPM significantly increased the encapsulation rate of LA into nanoparticles. FIG. 11B illustrates the maximum LA loading in samples containing Ionic Liquid 1, 2, or 4. Ionic Liquids 1, 2, and 4 were able to significantly increase the loading of LA into nanoparticles.

(Skin Penetration Test)

The compositions listed in Table 3 were subjected to a skin permeation test using a Franz diffusion cell in which mouse skin was placed. Comparative Example 5 contains diethylene glycol monoethyl ether (DGME), a chemical penetration enhancer, as a surfactant. A piece (2 × 2 $cm^2$) of mouse skin (Hos-HR-1, Hoshino Laboratory Animals, Inc.) was placed in a Franz diffusion cell filled with 5 mL of HEPES buffer (HEPES salt at 31 M concentration in MilliQ and adjusted to pH 7.4 with NaOH and HCl solution) in a receiver chamber. 250 μL of the composition was added to a shaved mouse skin portion to be used as a donor compartment, the system was maintained at 32.5°C using a circulating water bath, and the receiver phase was stirred by rotation of a stirrer via magnetic force.

At 1, 3, 6, 9, 24, and 36 hours, the composition (300 μL) was added to the donor compartment while 300 μL of media was aspirated to replace the receiver phase. Transdermal (through the skin) and topical (in the skin) LA were quantified by HPLC. For the determination of LA in the skin, the donor compartment was unfixed after 36 hours, and the skin surface was washed a plurality of times with a 20% ethanol solution. The skin was cut into 16 pieces and LA was extracted by agitation in dilute solution for 12 hours. The extracted solution was diluted from 10 to 100 times and the concentration of LA was determined by HPLC.

[Table 3]

| | Surfactant 5% (w/w) | Auxiliary surfactant 5% (w/w) | Basic liquid 1mL | LA |
|---|---|---|---|---|
| Example 1 | [EDMPC][Lin] | span-20 | IPM | 3mg/mL |
| Example 2 | [EDMPC][Ole] | span-20 | IPM | 3mg/mL |
| Example 3 | [EDMPC][Ste] | span-20 | IPM | 3mg/mL |
| Comparative Example 5 | DGME | span-20 | IPM | 3mg/mL |
| Comparative Example 6 | tween-80 | span-20 | IPM | 3mg/mL |
| Comparative Example 7 | [EDMPC][Lin] | span-20 | PBS | 3mg/mL |
| Comparative Example 8 | - | - | PBS | 3mg/mL |

As illustrated in FIG. 12, the concentration of LA in the receiver phase was highest after 24 hours and decreased gradually, except for Example 1. In Example 1, the concentration of LA increased even after 36 hours. FIG. 13 illustrates the amount of LA in the transdermal and topical areas after 36 hours. The highest amount of LA was penetrated into the skin and transported transdermally by Example 1.

Skin penetration kinetic parameters were evaluated by lag time and least squares method. The dermal flux (J) and penetration coefficient (Kp) were determined based on Kp = J/Cd. Cd is the concentration of LA in a donor preparation (μg/mL). Lag time ($t_L$) was determined from the intercept on the x-axis, and diffusion coefficient (D) was determined from D = $l^2/6\ t_L$. l is the thickness of mouse skin 0.0041 cm. The skin partition coefficient ($K_{skin}$) was calculated from $K_{skin}$ = (J × l)/(D × Cd). Skin penetration kinetic parameters are listed in Table 4.

[Table 4]

| | Cumulative amount $Q_{36h}$ (μg/$cm^2$) | Transdermal flux J (μg/$cm^2$/h) | Penetration coefficient $K_p$ ($10^{-4}$ cm/h) | Diffusion coefficient D ($10^{-4}$cm/h) | Skin partition coefficient $K_{sin}$ (×$10^{-2}$) |
|---|---|---|---|---|---|
| Example 1 | 427 | 0.25 | 5.63 | 2.16 | 14.49 |
| Example 2 | 2.78 | 0.17 | 4.04 | 1.98 | 12.35 |
| Example 3 | 2.99 | 0.21 | 5.13 | 2.02 | 13.22 |
| Comparative Example 5 | 1.51 | 0.13 | 3.41 | 1.63 | 10.71 |
| Comparative Example 6 | 1.92 | 0.15 | 3.54 | 1.29 | 11.96 |
| Comparative Example 7 | 1.71 | 0.09 | 2.65 | 1.11 | 9.73 |
| Comparative Example 8 | 0.14 | 0.02 | 0.51 | 0.51 | 3.83 |

(Influence of IL/O-ND on Stratum Corneum of Skin)

Frozen skin of a pig (YMPC, Hoshino Test Animal Breeding Co., Ltd.) was returned to room temperature, dried skin was warmed at 60°C for from 60 to 120 seconds, and the epidermal layer was peeled from the skin. The collected epidermal sheets were drifted in 0.25% trypsin and 1 mM ethylenediaminetetraacetic acid (EDTA) solution with the stratum corneum side facing up for 24 hours at room temperature. The isolated stratum corneum was washed with water and dried for an additional 24 hours. Sections of stratum corneum were immersed in test samples (IPM, Ionic Liquids 1, 2, 4, tween-80, DGME or PBS) in glass tubes for 30 minutes at room temperature. Stratum corneum sheets were washed with 20% ethanol and dried for 1 hour. The stratum corneum sheets were analyzed by Fourier transform infrared spectroscopy (FTIR) spectroscopy. Untreated stratum corneum was used as a control.

The lipid matrix organization (cholesterol, fatty acids and ceramides in lamellar structures) and the keratinous protein structure of the stratum corneum are the main barriers to transdermal DDS and affect the rate of drug diffusion into the deeper layers of skin. FTIR spectra of the stratum corneum indicated absorption of lipid stretching regions at from 2,825 to 2,975 $cm^{-1}$ and protein amide structures at 1,475 to 1,725 $cm^{-1}$. Influence of II,/O-ND on the skin layer is listed in Table 5. In the spectra, the absorption of amide structures or keratin proteins exhibits shifts of 1,550 $cm^{-1}$ with respect to amide-I; -C=O and 1,650 $cm^{-1}$ with respect to amide-II; NH-C=O, and the shifts of 2,845 $cm^{-1}$ and 2,923 $cm^{-1}$ correspond to carbohydrates of the vibrational stretching of lipids pertaining to $CH_2$ symmetry and $CH_2$ asymmetry. The disassembly of the stratum corneum is directly related to the molecular diffusion of a drug through the skin. The deformability of the $\alpha$-helix colloidal and $\beta$-sheet structures of keratinous proteins is promoted and drug penetration is enhanced. These shifts are promoted in ionic liquids because of the lipophilic cations and fatty acid anions of the ionic liquids.

The carbons of the unsaturated double bonds of linoleic acid (C18:2) in Ionic Liquid 1 have a considerable influence on accelerated deformation of hydrogen bonds in the stratum corneum. These shifts are associated with the Gauche/trans conformation that characterizes the organization of lipids in the stratum corneum, suggesting reduced lipid barrier function. The largest $CH_2$ symmetric and $CH_2$ asymmetric shifts in Ionic Liquid 1 indicate that Ionic Liquid 1 efficiently impairs barrier function and is suitable for transdermal DDS.

[Table 5]

| | Stratum corneum layer component (lipid) | | | | | | Stratum corneum component (protein) | | | | | |
|---|---|---|---|---|---|---|---|---|---|---|---|---|
| | Absorbance; $CH_2$ asymmetric (crti 1) | ⊿shift ($cm^{-1}$) | ⊿shift (intensity) | Absorbance; $CH_2$ symmetric ($cm^{-1}$) | ⊿shift ($cm^{-1}$) | ⊿shift (intensity) | Absorbance; C=O amide -II ($cm^{-1}$) | ⊿shift ($cm^{-1}$) | ⊿shift (intensity) | Absorbance; NH-C=O amide -I ($cm^{-1}$) | ⊿shift ($cm^{-1}$) | ⊿shift (intensity) |
| Control | 2918 | - | 0.00 | 2851 | - | 0.00 | 1647 | - | 0.00 | 1537 | - | 0.00 |
| PBS | 2919 | 1 | 1.80 | 2852 | 1 | 006 | 1648 | 1 | 802 | 1538 | 1 | 7.80 |
| IPM | 2920 | 2 | 172 | 2853 | 2 | 108 | 1648 | 1 | 13.95 | 1539 | 2 | 10.59 |
| DGME | 2918 | 0 | 2.37 | 2853 | 2 | 1.25 | 1648 | 1 | 1800 | 1541 | 4 | 1431 |
| tween-80 | 2920 | 2 | 280 | 2853 | 2 | 1.30 | 1648 | 1 | 1901 | 1542 | 5 | 16.14 |
| Ionic Liquid 4 | 2921 | 3 | 276 | 2853 | 2 | 260 | 1648 | 1 | 1906 | 1542 | 5 | 17 32 |
| Ionic Liquid 2 | 2919 | 2 | 453 | 2853 | 2 | 266 | 1650 | 2 | 22.15 | 1541 | 4 | 21.89 |
| Ionic Liquid 1 | 2922 | 4 | 5.17 | 2854 | 3 | 299 | 1649 | 1 | 23.81 | 1545 | 8 | 2222 |

(Effect of IL/O-ND on Pharmacokinetics)

Pharmacokinetic studies of IL/O-ND were performed in BALB/C mice (female, 6 weeks old, 20 ± 2 g, from KYUDO CO., LTD.) randomly divided into 6 groups of 5 mice per group. The hair on the back of the mice was removed, and after 2 days, 300 μL of preparation listed in Table 6 (90 μg LA/mouse) per mouse was administered to clean skin using a 1 cm × 1 cm patch. Mice injected subcutaneously with 300 μL of PBS containing 90 μg LA served as positive controls. After a predetermined time period, approximately 200 μL of blood from the posterior orbit was collected from the eyes of the mice. Blood samples were also collected from the injected group after a predetermined time lapse. Serum was obtained by centrifuging the blood samples at 10,000 rpm for 20 minutes. The concentration of LA in the plasma was determined by enzyme-linked immunosorbent assay (ELISA) as follows.

100 μL of plasma with 4% phosphoric acid was vortexed and applied to a WCX-SPE column. The WCX SPE column was washed with 200 μL of 5% ammonium hydroxide followed by 20% acetonitrile, and LA was eluted with 100 μL of acetonitrile/water (75/25) solution containing 1% TFA. The eluted LA was evaporated in a microevaporator, eluted in 100 μL of water, and evaluated by ELISA according to the protocol of LHRH (Leuprolide) ELISA kit (A18102, BMA Biomedicals, Peninsula laboratories). The concentration of LA in plasma was determined by measuring the absorbance of the sample at 450 nm.

FIG. 14 illustrates changes over time in the concentration of LA in plasma. Table 6 provides pharmacokinetic parameters. Transdermal administration of LA was better than injection. With injection, the concentration of LA in plasma increased significantly at 30 minutes post-dose and decreased after 4 hours. On the other hand, transdermal administration, especially with Example 1, gradually increased the concentration of LA in plasma up to 36 hours after administration. Example 1 was found to be suitable for transdermal DDS using the patch.

[Table 6]

| Preparation | Example 1 (transdermal) | Comparative Example 6 (transdermal) | Comparative Example 5 (transdermal) | Comparative Example 8 (transdermal) | Comparative Example 8 (injection) |
|---|---|---|---|---|---|
| Surfactant (5%w/w) | [EDMPC][Lin] | tween-80 | DGME | - | - |
| Auxiliary surfactant (5%w/w) | span-20 | span-20 | span-20 | - | - |
| Basic liquid (lmL) | IPM | IPM | IPM | PBS | PBS |
| Concentration of LA (mg/mL) | 3 | 3 | 3 | 3 | 3 |
| $AUC_{0-t}$ (pg · hr/mL) | 85.58 | 67.67 | 64.01 | 522 | 28.21 |
| $C_{max}$ (pg/mL) | 5.91 | 4.61 | 4.22 | 1.72 | 4.81 |
| $C_{max}$ time (h) | 36 | 24 | 12 | 9 | 4 |
| Constant rate K (h-1) | 0.01 | 0.06 | 0.07 | 0.18 | 0.75 |

(Evaluation of Biocompatibility)

MTT cell viability assays were performed as described above for the preparations listed in Table 7. In Table 7, e-TFSA indicates 1-dodecyl-3-methylimidazolium bis(trifluoromethylsulfonyl)imide, and e-TF2N indicates 1-dodecyl-3-methylimidazolium bis(trifluoroethylsulfonyl)imide.

In addition, the in vivo biocompatibility of each preparation was evaluated using female BALB/C mice (from KYUDO CO., LTD.). Preparations at 300 μL/mouse were administered through the skin via a patch three times with an interval of 5 days. For each dose, the patch was maintained on the skin for 24 hours. Body weights were measured every other day until the last day, and at the end of the experiment, skin samples were taken for histological analysis. For histological sample preparation and staining, skin was exposed to water and 50% 2-propanol, then frozen in 4% formaldehyde solution at -30°C for from 2 to 3 hours. Sections approximately 20 μm thick were obtained from the skin and fixed to slides. Sections were washed with acetone, ethanol, and water, and unwanted residues were removed. Sections were stained with hematoxylin solution for 6 hours, washed with water, and stained with eosin solution for

another 20 to 30 minutes. After washing with water and dehydration with ethanol, sections were covered with glass and observed under a color microscope (BZ-9000, manufactured by KEYENCE CORPORATION).

[Table 7]

| Preparation | Surfactant (5% wt./V) | Auxiliary surfactant (5% wt./V) | Basic liquid |
|---|---|---|---|
| Comparative Example 8 | - | - | PBS |
| Comparative Example 9 | - | - | IPM |
| Example 1 | [EDMPC][Lin] | span-20 | IPM |
| Comparative Example 6 | tween-80 | span-20 | IPM |
| Comparative Example 5 | DGME | span-20 | IPM |
| Comparative Example 10 | e-TFSA | span-20 | IPM |
| Comparative Example 11 | e-$TF_2N$ | span-20 | IPM |
| Comparative Example 12 | SDS | - | PBS |

FIG. 15 illustrates the cell viability. In addition to Comparative Examples 6, 8, and 9, the cell viability in Example 1 was close to 100%. In contrast, Comparative Examples 5, 10, 11, and 12 had viabilities of 59%, 53%, 18%, and 6%, respectively. These results indicated the *in vitro* biocompatibility of Example 1.

FIG. 16 illustrates the body weights of the mice measured every other day. In all cases, there was no considerable change in body weight until 15 days, but in Comparative Example 11, the mice died after the first dose, and their skin was darkened and destroyed. FIG. 17 illustrates the stratum corneum imaged at 20× magnification by fluorescence microscopy. No damage was observed in the stratum corneum of untreated, Example 1, and Comparative Examples 6 and 9. In Comparative Examples 5 and 10, slight damage was observed, and in Comparative Examples 11 and 12, damage was observed in both layers of skin. These results indicate that Example 1 is biocompatible and safe in vivo and is therefore useful for pharmaceutical preparations, especially transdermally absorbable agents.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

This application claims the benefit of Japanese Patent Application No. 2020-056457, filed on March 26, 2020, the entire disclosure of which is incorporated by reference herein.

Industrial Applicability

The ionic liquid according to the present disclosure has low toxicity and excellent biocompatibility, and that exhibits high solubility for both a hydrophilic substance and a hydrophobic substance, and therefore, can be safely used in a variety of fields, including biotechnology-related fields. Accordingly, the invention has high industrial applicability.

## Claims

**1.** An ionic liquid having a structure represented by the following general formula (1).
[Chem. 1]

General formula (1) $R\text{-}COO^{-}\ X^{+}$

[In the general formula (1), R represents a substituted or unsubstituted alkyl group or a substituted or unsubstituted alkenyl group, and at least one ethylene group comprising the alkenyl group may be substituted with a vinylene group. $X^+$ represents a phospholipid with a cationic group.].

**2.** The ionic liquid according to claim 1, wherein $X^+$ in the general formula (1) is a glycerophospholipid with a cationic group.

**3.** The ionic liquid according to claim 2, wherein $X^+$ of the general formula (1) has a structure represented by the

following general formula (2).

[Chem. 2]

General formula (2)

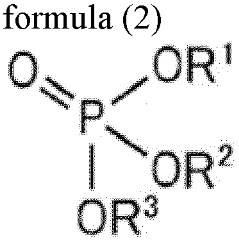

[In the general formula (2), $R^1$ represents an alkyl group substituted with a cationic group, $R^2$ represents a hydrogen atom or a substituted or unsubstituted alkyl group, and $R^3$ represents a substituted or unsubstituted alkyl group.].

**4.** The ionic liquid according to claim 3, wherein $R^3$ in the general formula (2) is an alkyl group substituted with an alkyl carbonyloxy group.

**5.** The ionic liquid according to claim 4, wherein $R^3$ in the general formula (2) is an alkyl group substituted with two or more alkyl carbonyloxy groups.

**6.** The ionic liquid according to any one of claims 3 to 5, wherein $R^2$ in the general formula (2) is a substituted or unsubstituted alkyl group.

**7.** The ionic liquid according to any one of claims 1 to 6, wherein the cationic group is a quaternary ammonium group.

**8.** The ionic liquid according to claim 2, wherein $X^+$ in the general formula (1) is a derivative of phosphatidylcholine.

**9.** The ionic liquid according to any one of claims 1 to 8, wherein the number of carbon atoms of R in the general formula (1) is from 8 to 22.

**10.** The ionic liquid according to any one of claims 1 to 9, wherein R in the general formula (1) contains an unsaturated bond.

**11.** The ionic liquid according to claim 10, wherein R in the general formula (1) has a polyene structure.

**12.** The ionic liquid according to any one of claims 1 to 11, wherein R in the general formula (1) is a group consisting only of carbon atoms and hydrogen atoms.

**13.** The ionic liquid according to any one of claims 1 to 12, that is hydrophobic.

**14.** The ionic liquid according to claim 13, that has a structure represented by the following formula.

[Chem. 3]

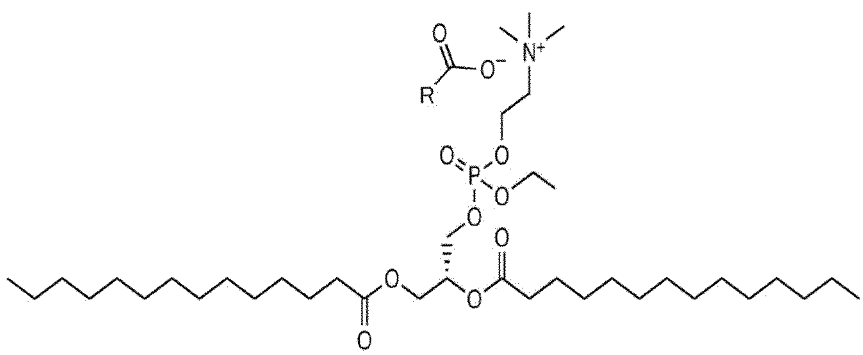

[In the formula, R represents a substituted or unsubstituted alkyl group or a substituted or unsubstituted alkenyl group, and at least one ethylene group comprising the alkenyl group may be substituted with a vinylene group.].

**15.** A solvent including the ionic liquid according to any one of claims 1 to 14.

**16.** A preparation including the ionic liquid according to any one of claims 1 to 14.

**17.** A transdermally absorbable agent including the ionic liquid according to any one of claims 1 to 14.

**18.** The transdermally absorbable agent according to claim 17, further including a sorbitan fatty acid ester.

**19.** The transdermally absorbable agent according to claim 18, wherein the sorbitan fatty acid ester is a sorbitan monolaurate.

**20.** The transdermally absorbable agent according to any one of claims 17 to 19, wherein R-$COO^-$ in the general formula (1) is a carboxylate ion in which a hydrogen ion is dissociated from the carboxy group of linoleic acid.

FIG. 1

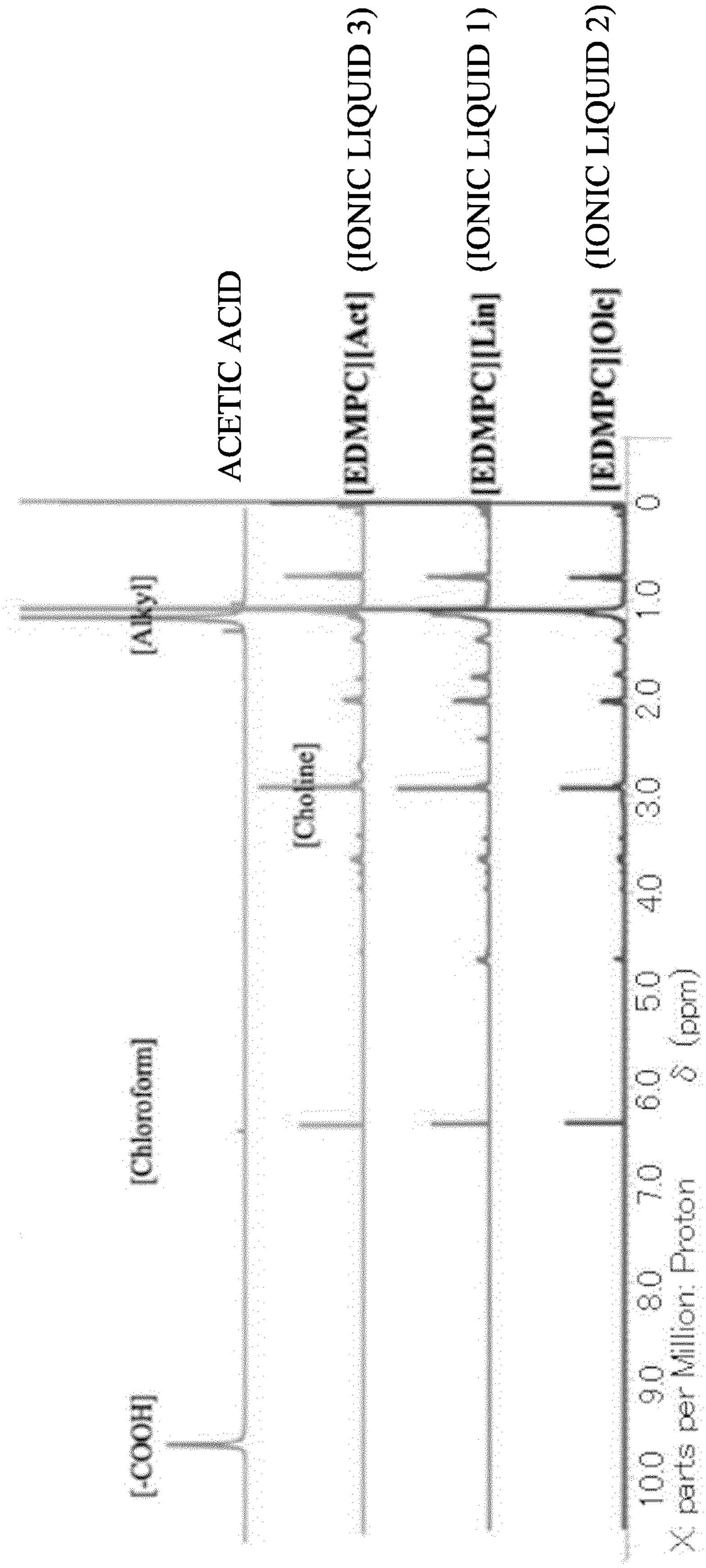
ACETIC ACID
[EDMPC][Act] (IONIC LIQUID 3)
[EDMPC][Lin] (IONIC LIQUID 1)
[EDMPC][Olc] (IONIC LIQUID 2)
[-COOH]
[Chloroform]
[Choline]
[Alkyl]
10.0 9.0 8.0 7.0 6.0 5.0 4.0 3.0 2.0 1.0 0
δ (ppm)
X: parts per Million: Proton

FIG. 2

LIQUID SAMPLE OF IONIC LIQUID 1 AND ISOPROPYL MYRISTATE

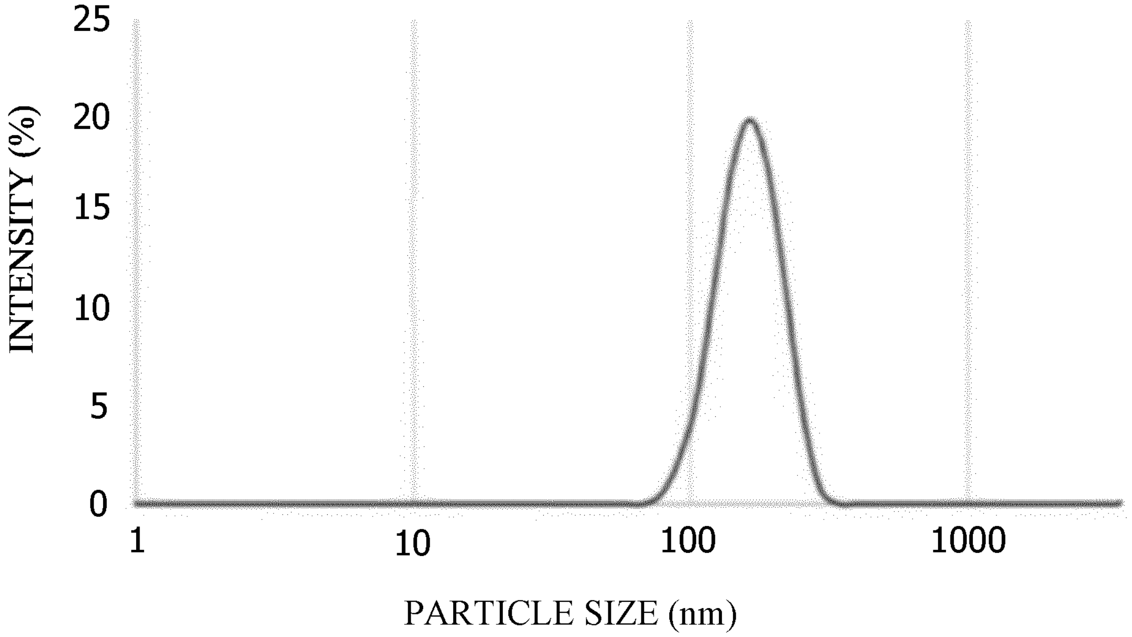


FIG. 3

LIQUID SAMPLE OF IONIC LIQUID 1 AND WATER

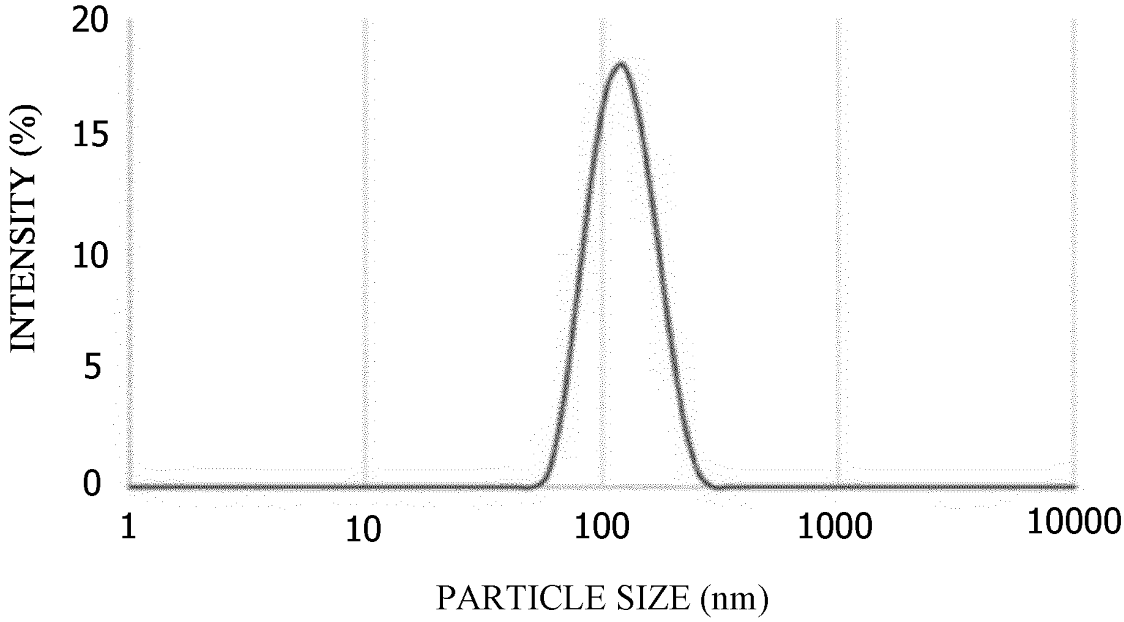

FIG. 4

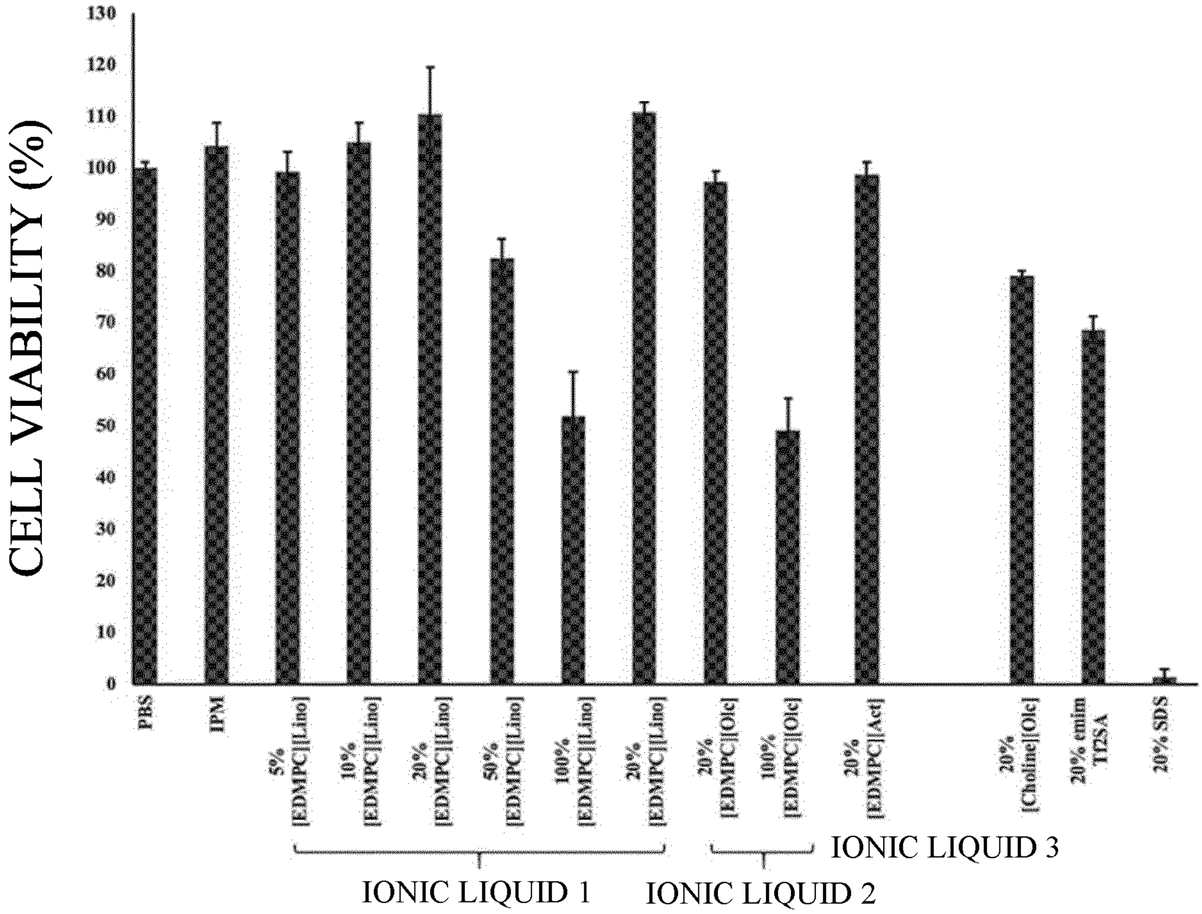
CELL VIABILITY (%)
130
120
110
100
90
80
70
60
50
40
30
20
10
0
PBS
IPM
5% [EDMPC][Lino]
10% [EDMPC][Lino]
20% [EDMPC][Lino]
50% [EDMPC][Lino]
100% [EDMPC][Lino]
20% [EDMPC][Lino]
20% [EDMPC][Olc]
100% [EDMPC][Olc]
20% [EDMPC][Act]
20% [Choline][Olc]
20% emim Tf2SA
20% SDS
IONIC LIQUID 1
IONIC LIQUID 2
IONIC LIQUID 3

FIG. 5A

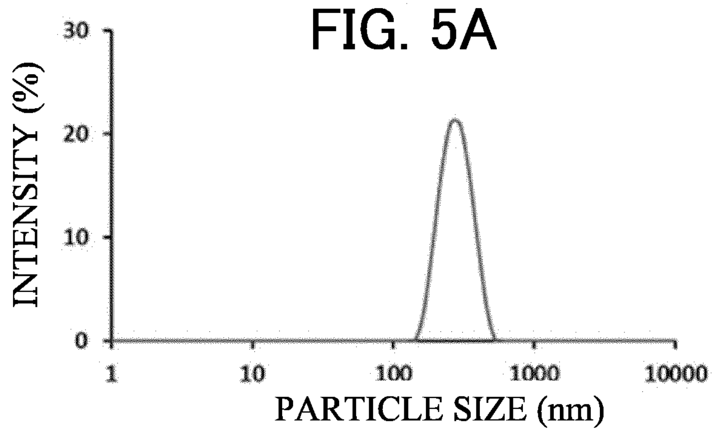


FIG. 5C

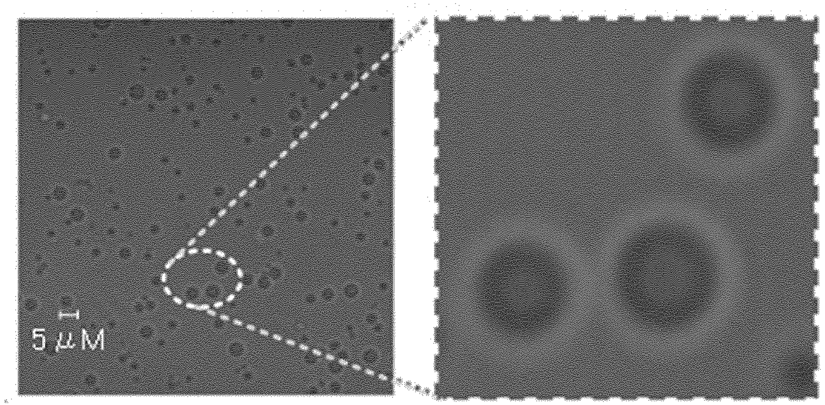


FIG. 5B

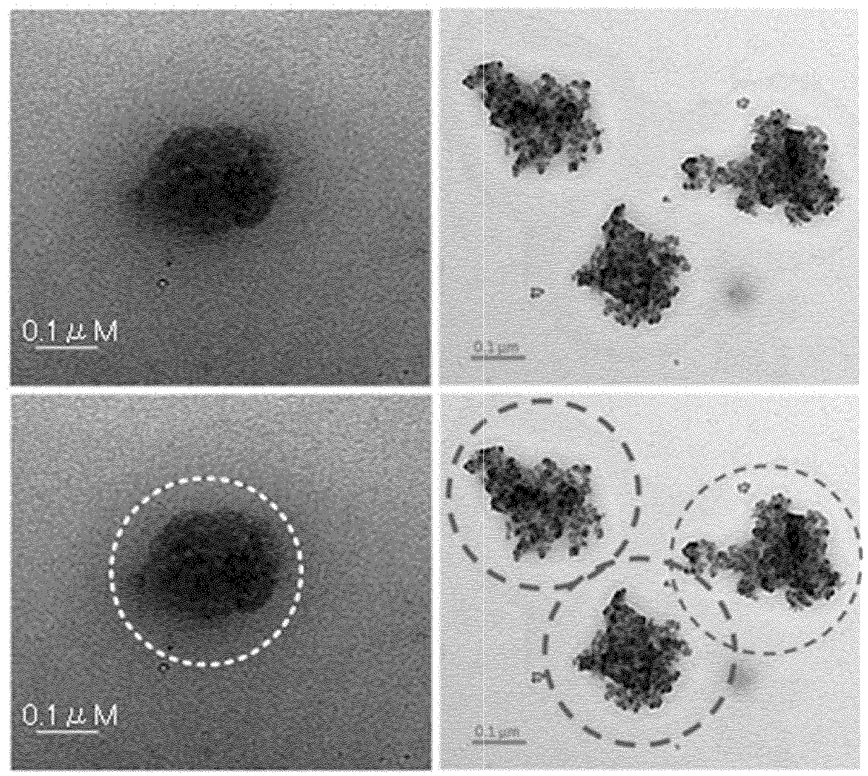

FIG. 6A

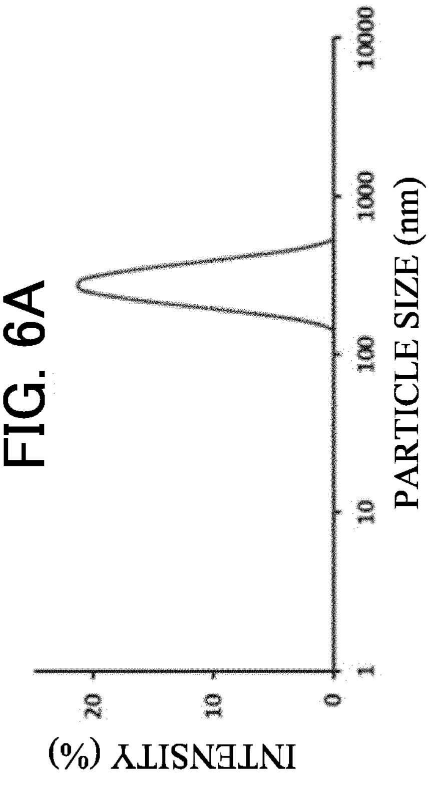


FIG. 6B

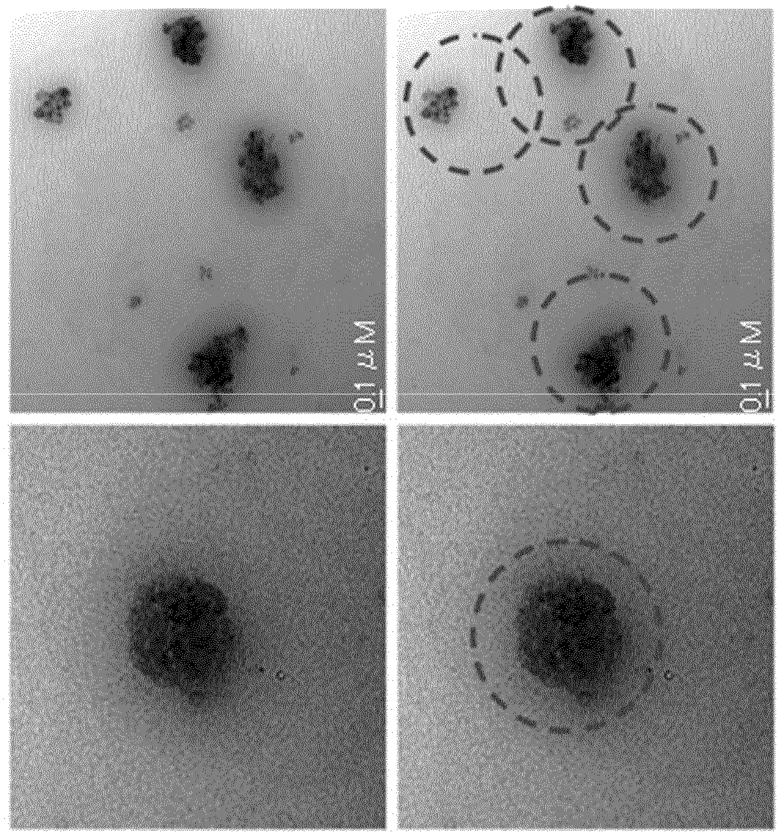


FIG. 6C

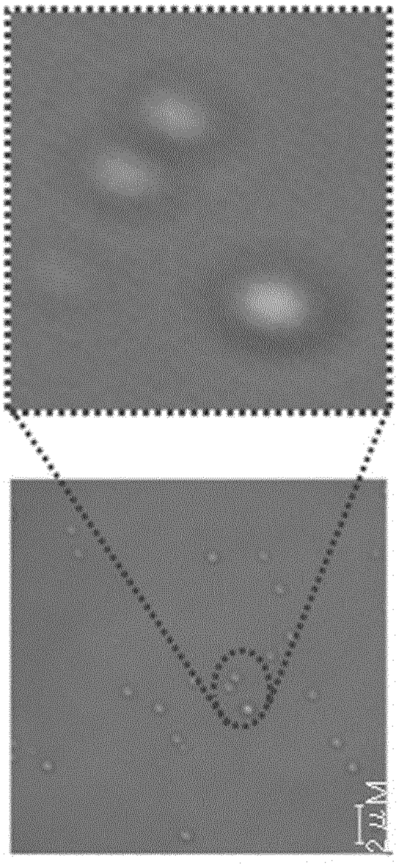

FIG. 7A

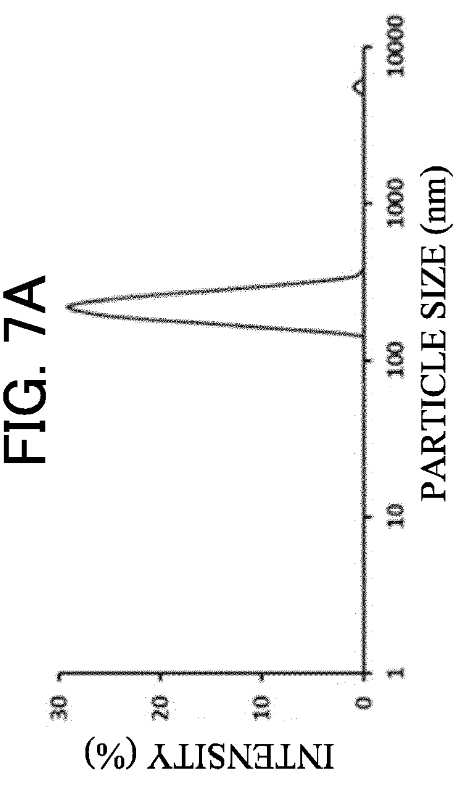


FIG. 7B

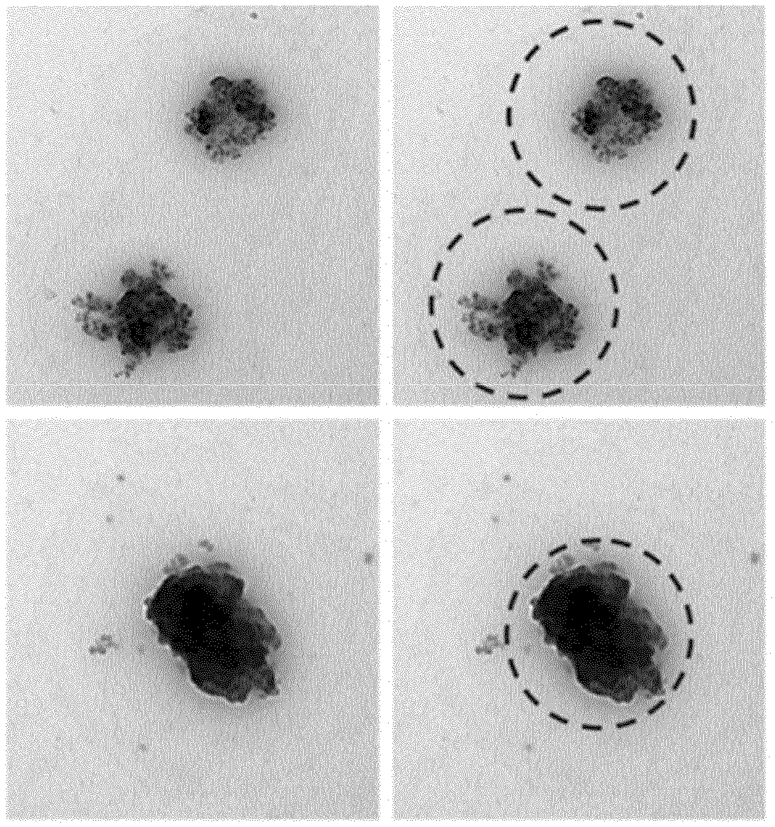

FIG. 7C

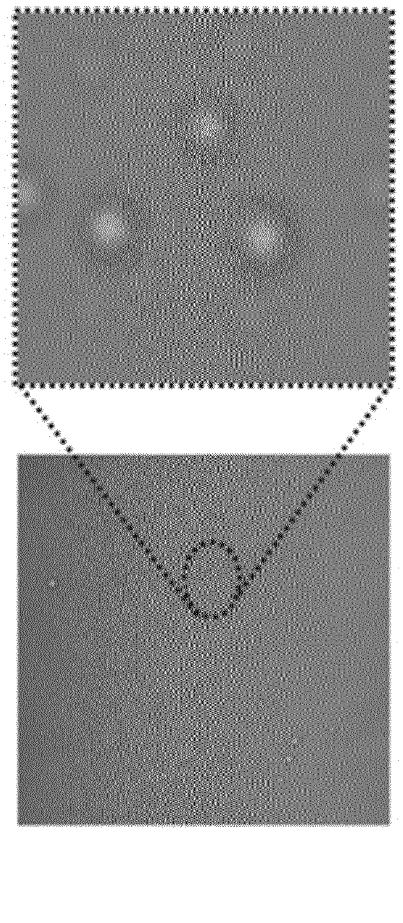

FIG. 8

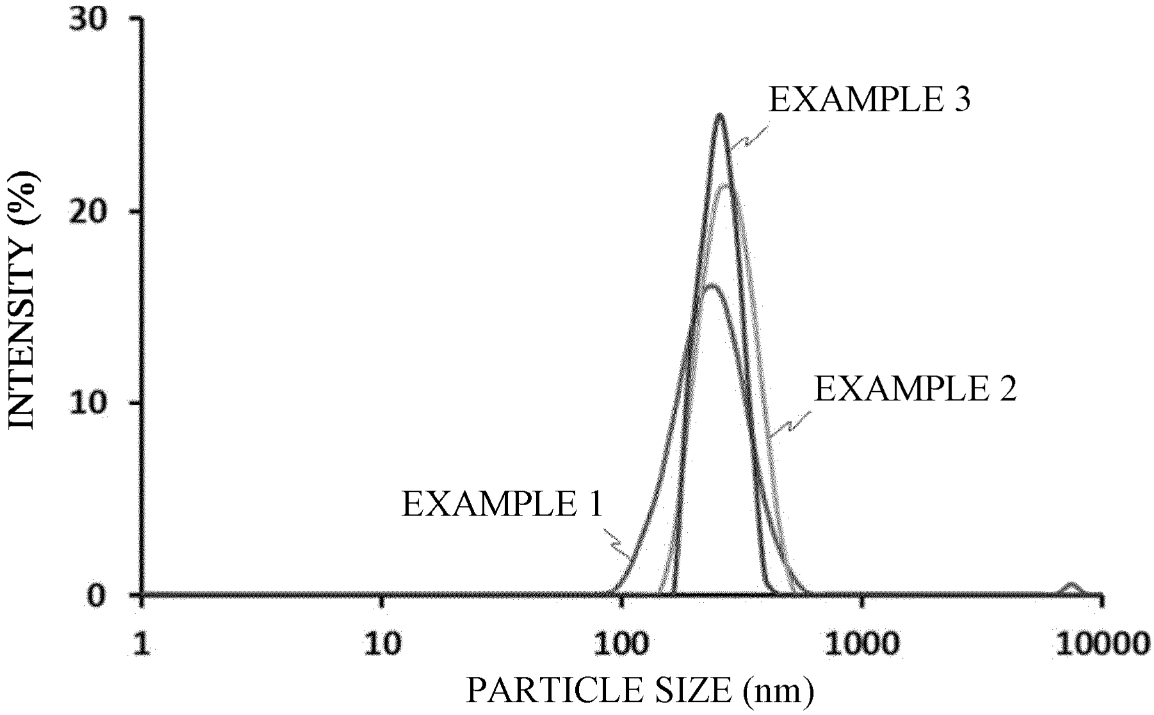
30
20
10
0
INTENSITY (%)
EXAMPLE 3
EXAMPLE 2
EXAMPLE 1
1
10
100
1000
10000
PARTICLE SIZE (nm)

FIG. 9B

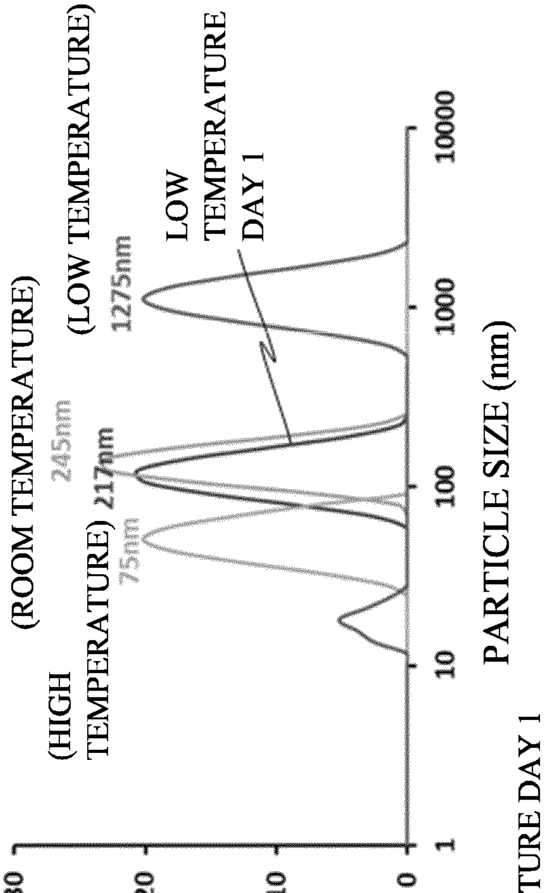


FIG. 9A

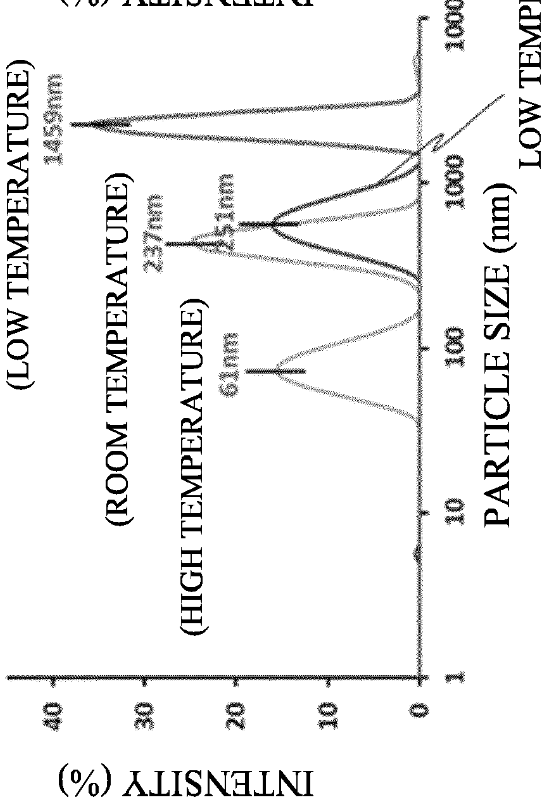


FIG. 9C

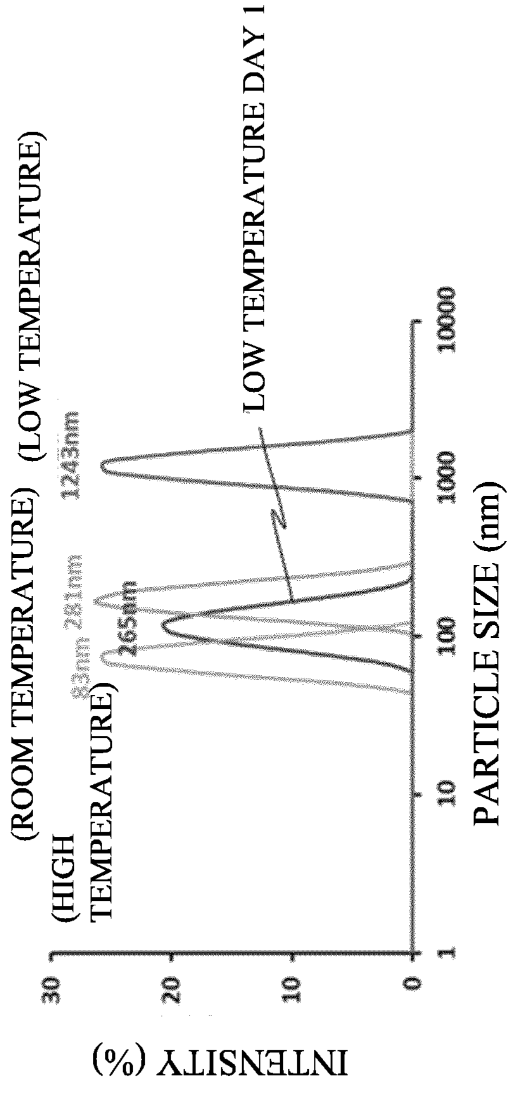

FIG. 10A

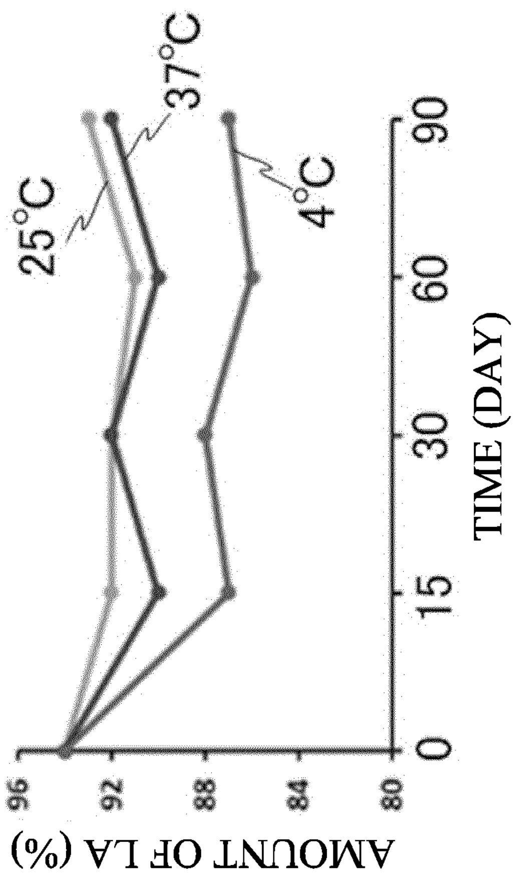


FIG. 10B

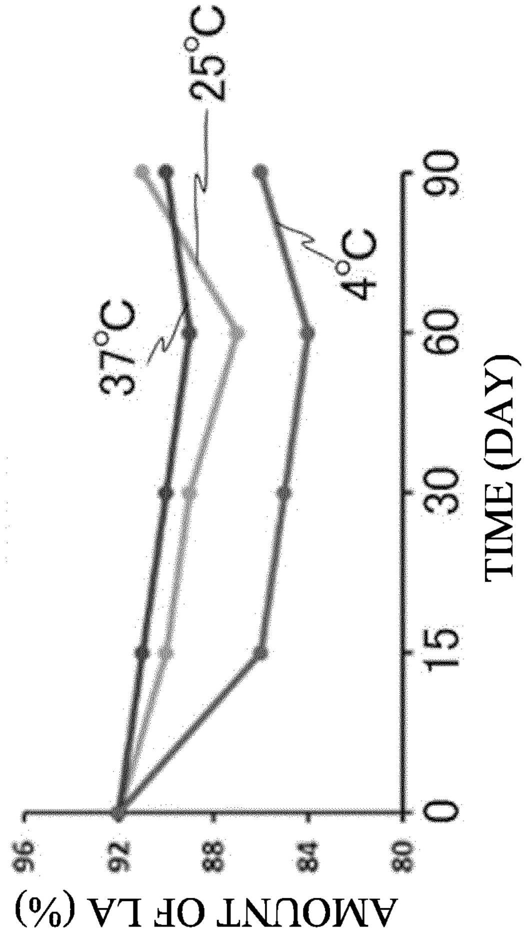


FIG. 10C

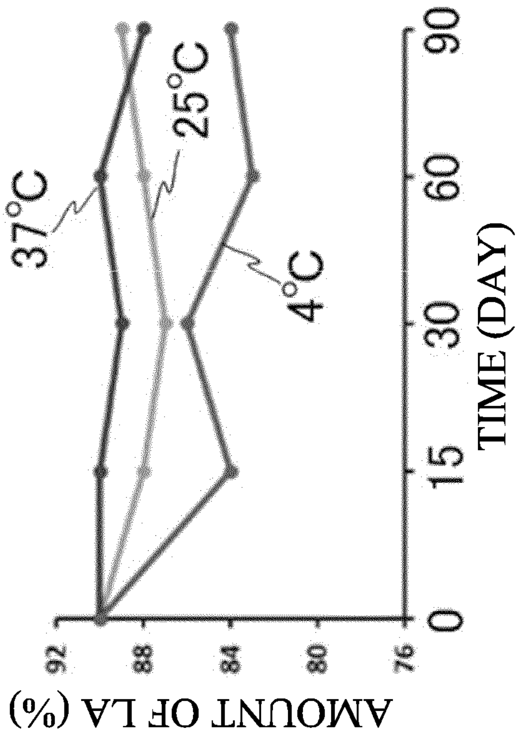

FIG. 11A

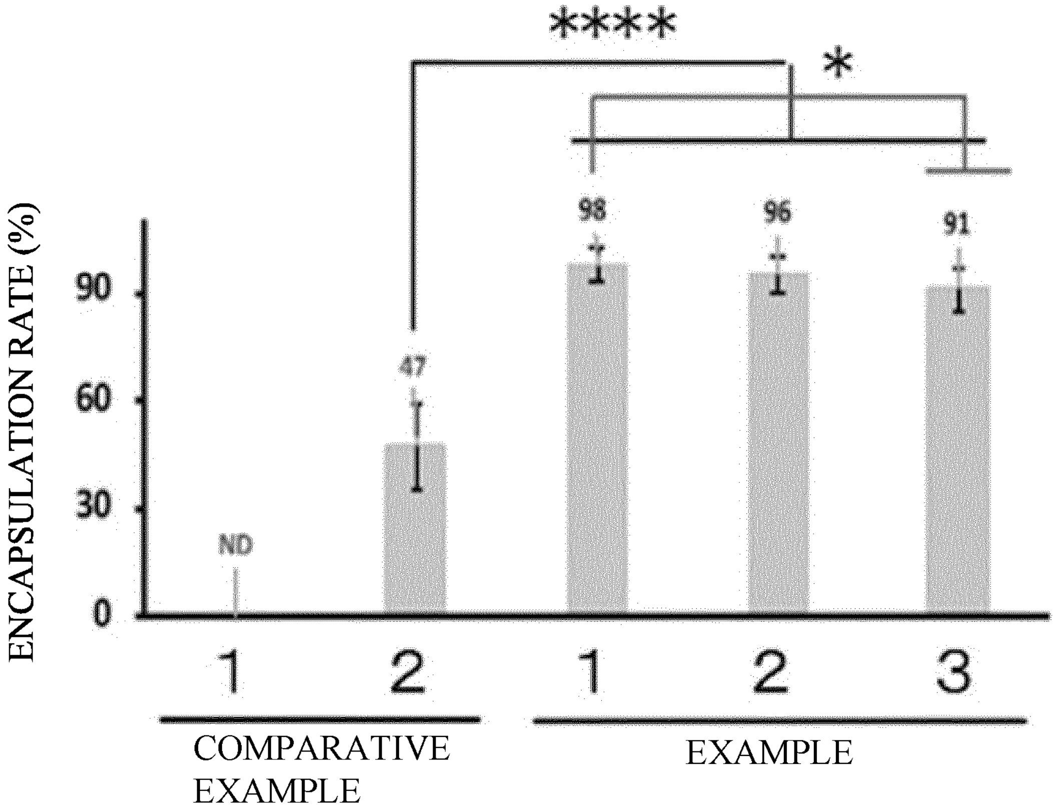


FIG. 11B

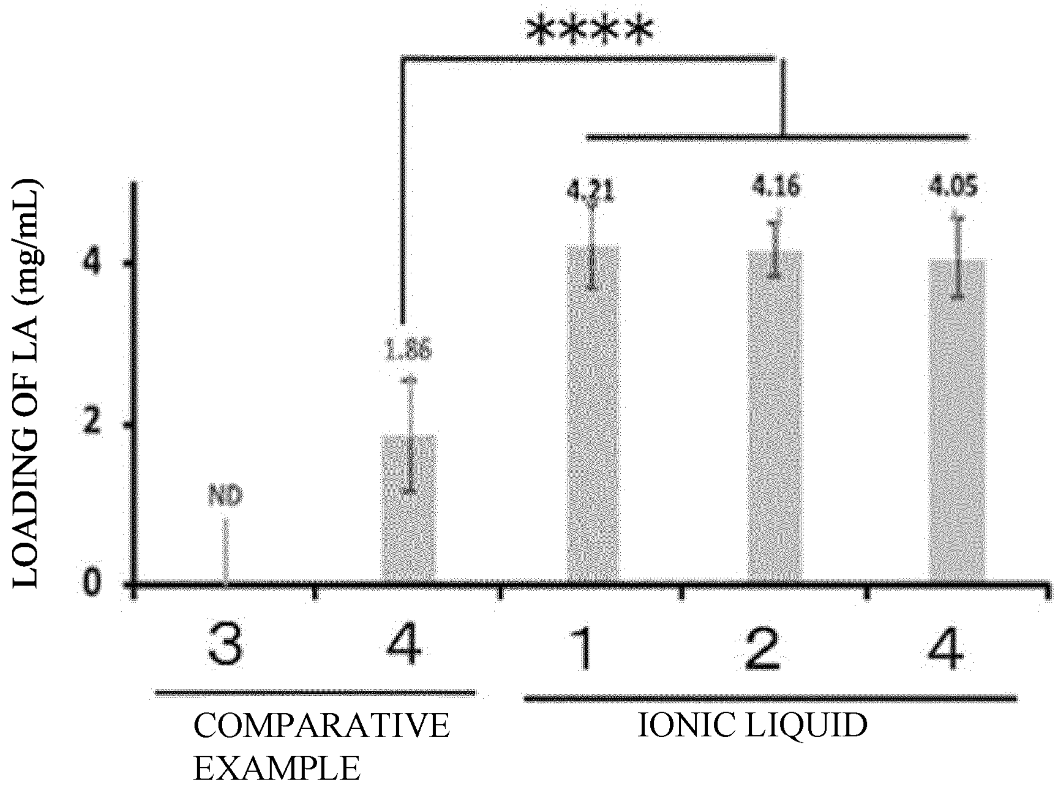

FIG. 12

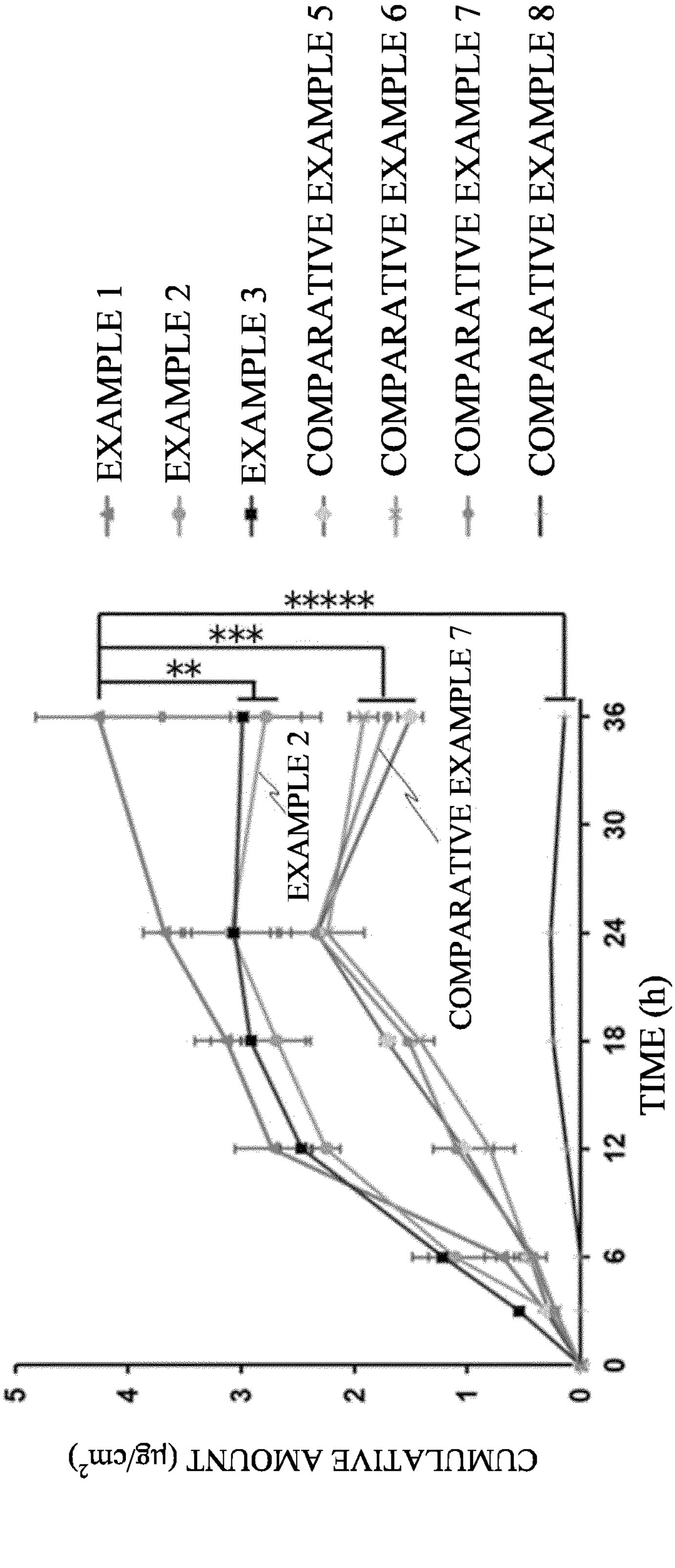
EXAMPLE 1
EXAMPLE 2
EXAMPLE 3
COMPARATIVE EXAMPLE 5
COMPARATIVE EXAMPLE 6
COMPARATIVE EXAMPLE 7
COMPARATIVE EXAMPLE 8
**
***
*****
EXAMPLE 2
COMPARATIVE EXAMPLE 7
CUMULATIVE AMOUNT (μg/cm2)
TIME (h)
0
1
2
3
4
5
0
6
12
18
24
30
36

FIG. 13

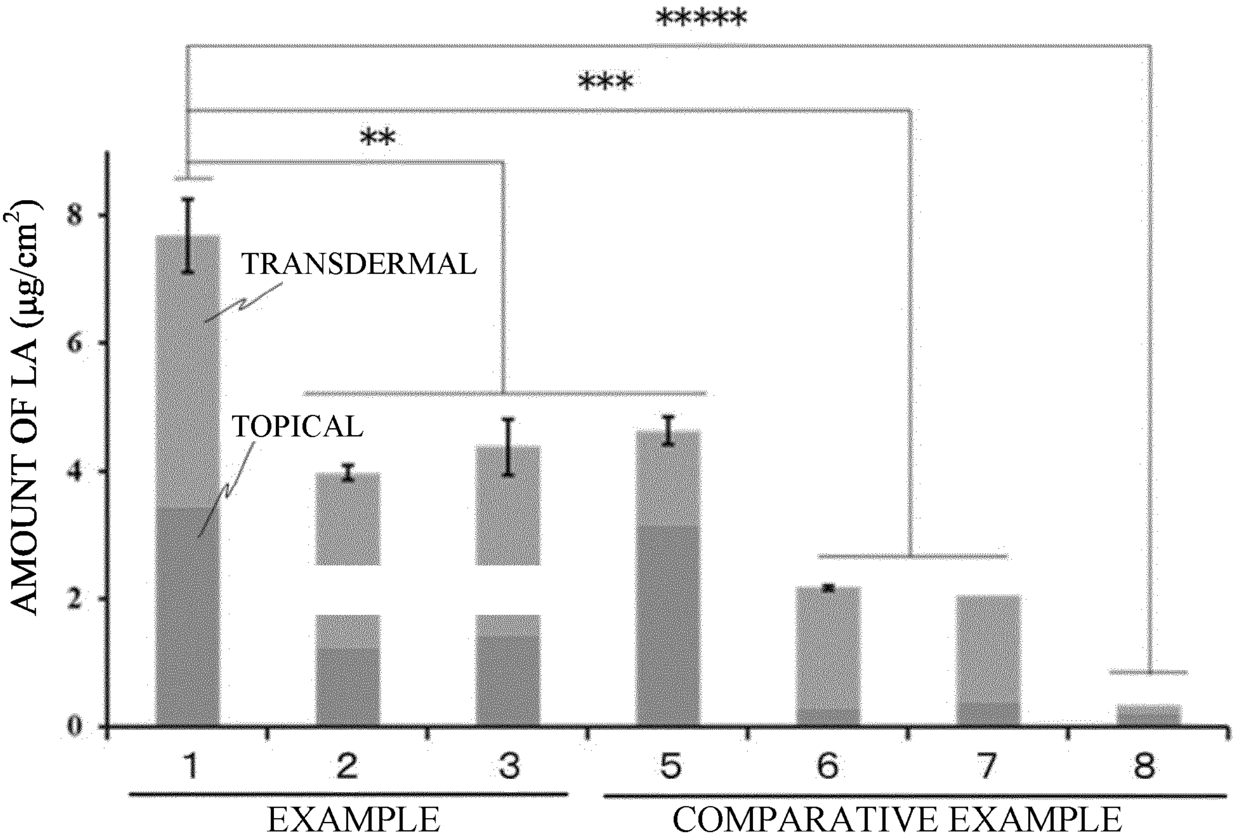
*****
***
**
AMOUNT OF LA (μg/cm2)
8
6
4
2
0
TRANSDERMAL
TOPICAL
1
2
3
5
6
7
8
EXAMPLE
COMPARATIVE EXAMPLE

FIG. 14

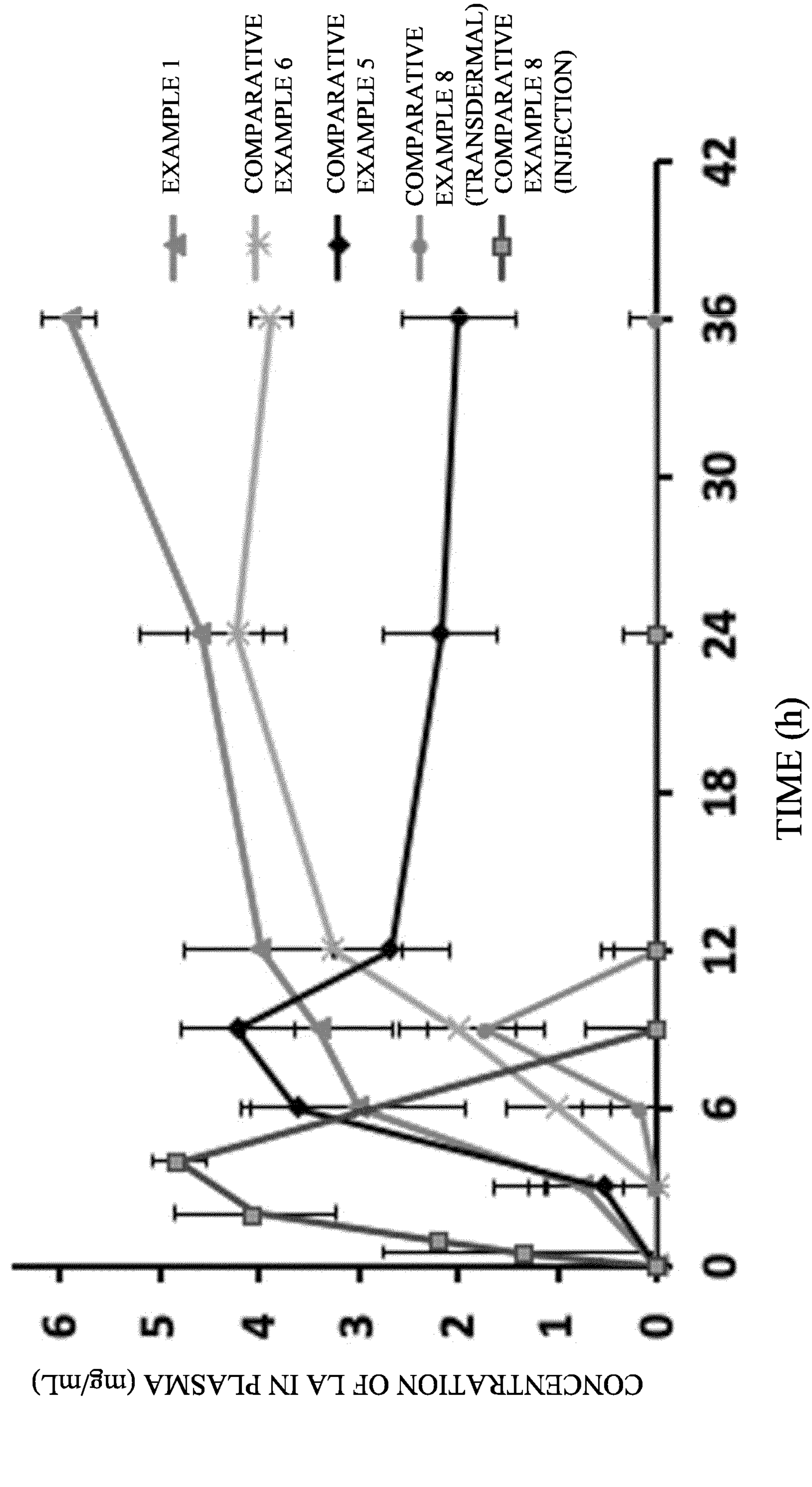

EXAMPLE 1
COMPARATIVE EXAMPLE 6
COMPARATIVE EXAMPLE 5
COMPARATIVE EXAMPLE 8 (TRANSDERMAL)
COMPARATIVE EXAMPLE 8 (INJECTION)
CONCENTRATION OF LA IN PLASMA (mg/mL)
0 1 2 3 4 5 6
TIME (h)
0 6 12 18 24 30 36 42

FIG. 15

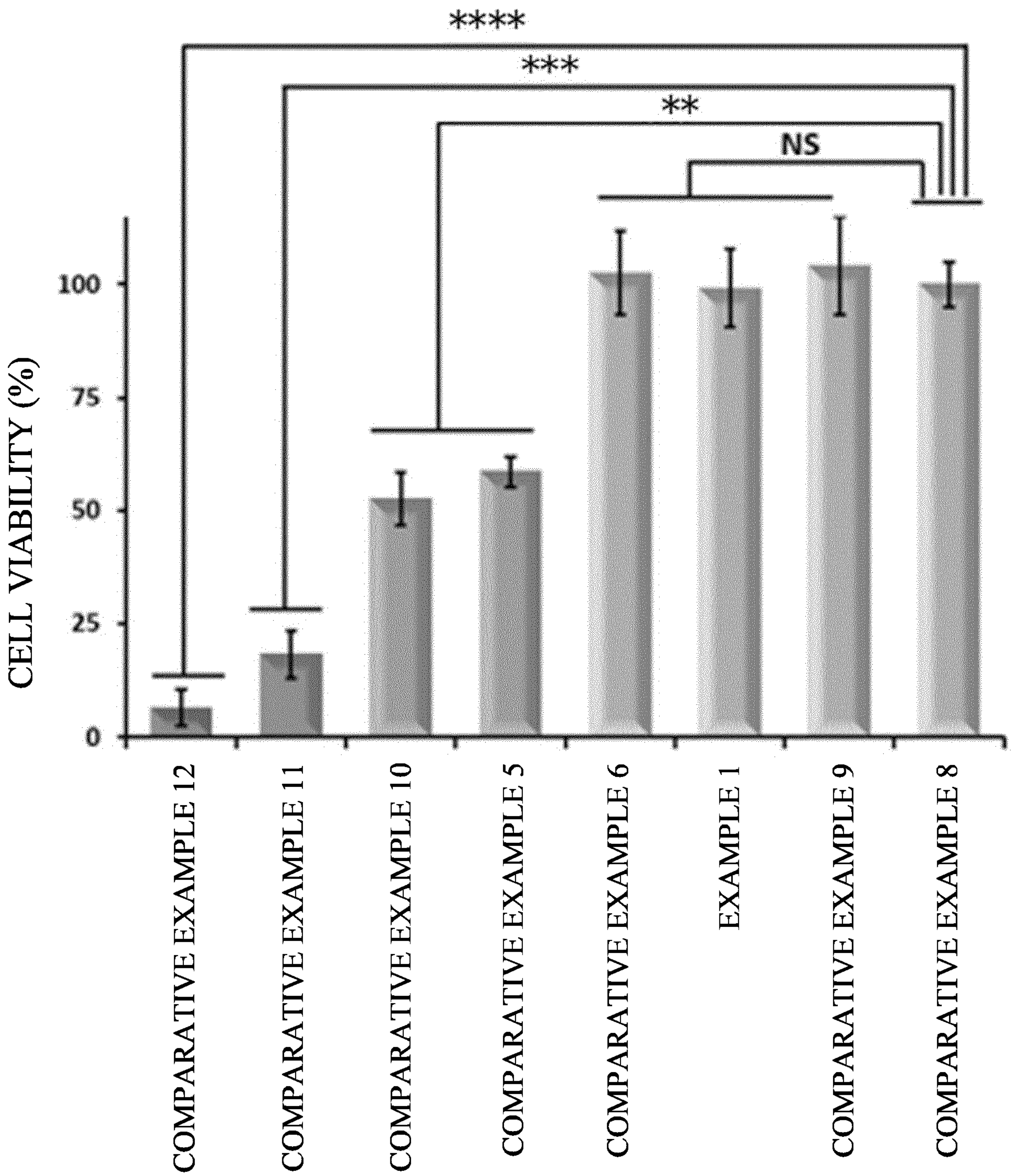
****
***
**
NS
CELL VIABILITY (%)
100
75
50
25
0
COMPARATIVE EXAMPLE 12
COMPARATIVE EXAMPLE 11
COMPARATIVE EXAMPLE 10
COMPARATIVE EXAMPLE 5
COMPARATIVE EXAMPLE 6
EXAMPLE 1
COMPARATIVE EXAMPLE 9
COMPARATIVE EXAMPLE 8

FIG. 16

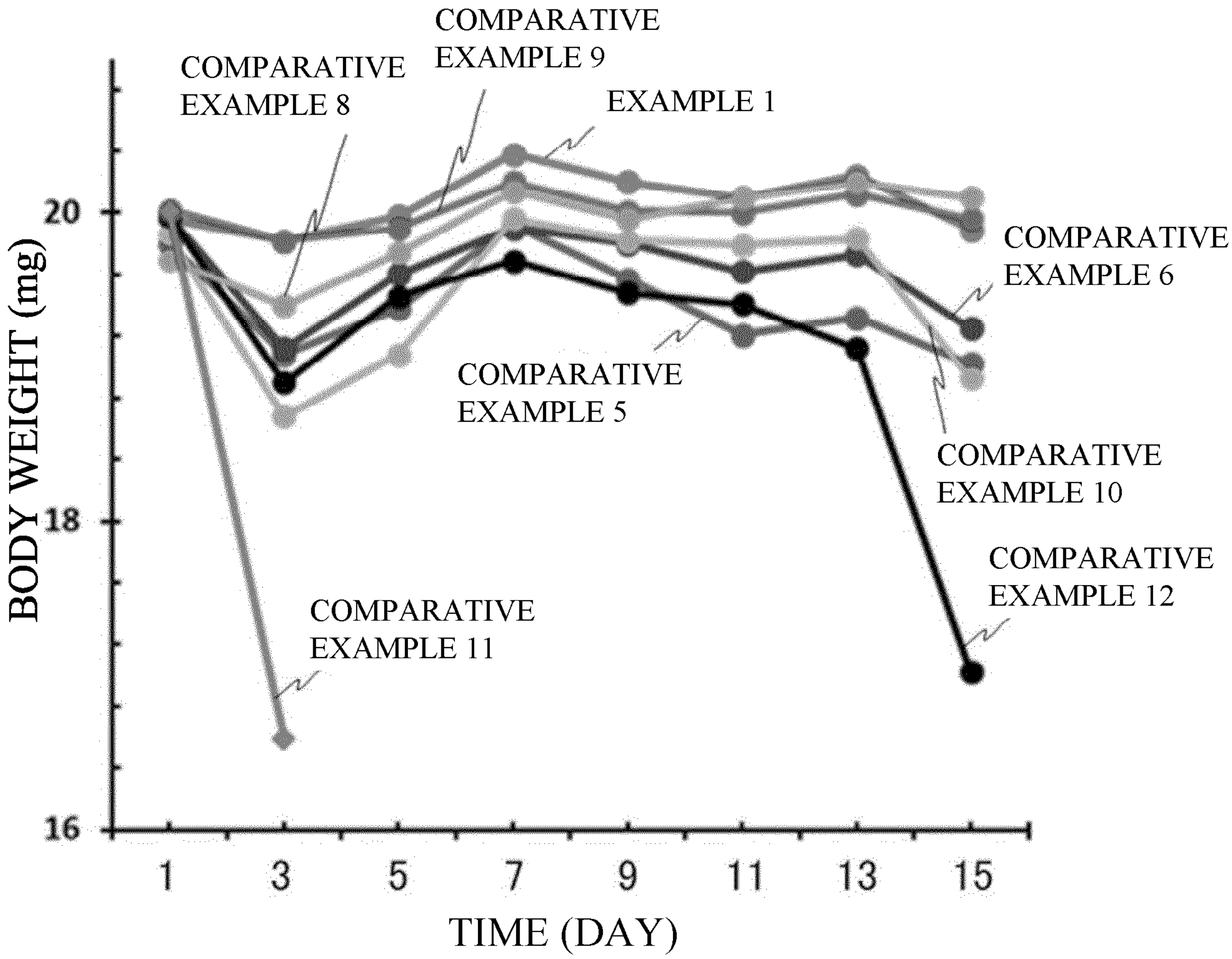
COMPARATIVE EXAMPLE 9
COMPARATIVE EXAMPLE 8
EXAMPLE 1
COMPARATIVE EXAMPLE 6
COMPARATIVE EXAMPLE 5
COMPARATIVE EXAMPLE 10
COMPARATIVE EXAMPLE 12
COMPARATIVE EXAMPLE 11
BODY WEIGHT (mg)
20
18
16
1
3
5
7
9
11
13
15
TIME (DAY)

FIG. 17

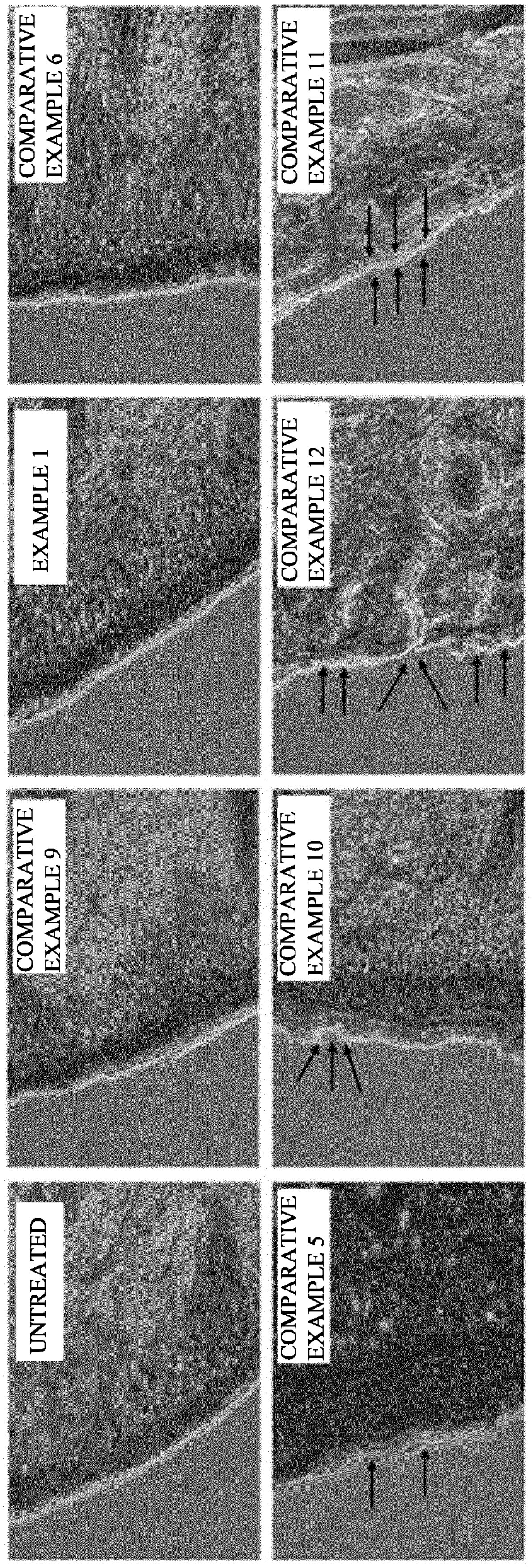
UNTREATED
COMPARATIVE EXAMPLE 9
EXAMPLE 1
COMPARATIVE EXAMPLE 6
COMPARATIVE EXAMPLE 5
COMPARATIVE EXAMPLE 10
COMPARATIVE EXAMPLE 12
COMPARATIVE EXAMPLE 11

**INTERNATIONAL SEARCH REPORT**

International application No.
PCT/JP2021/008074

A. CLASSIFICATION OF SUBJECT MATTER
Int. Cl. C07F9/09(2006.01)i, A61K9/08(2006.01)i, A61K47/12(2006.01)i, A61K47/24(2006.01)i, C07C53/08(2006.01)i, C07C53/126(2006.01)i, C07C57/12(2006.01)i
FI: C07F9/09 V, A61K9/08, A61K47/12, A61K47/24, C07C53/08, C07C53/126, C07C57/12

According to International Patent Classification (IPC) or to both national classification and IPC

B. FIELDS SEARCHED

Minimum documentation searched (classification system followed by classification symbols)
Int. Cl. C07F9/09, A61K9/08, A61K47/12, A61K47/24, C07C53/08, C07C53/126, C07C57/12

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII); CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2016/068336 A1 (ASAHI KASEI CORP.) 06 May 2016, claims, examples, paragraphs [0004]-[0009] | 1-20 |
| A | JP 2015-501328 A (ISIS INNOVATION LIMITED) 15 January 2015, claims, examples | 1-20 |
| A | MANINI, Paola et al. Nanoscale disassembly and free radical reorganization of polydopamine in ionic liquids, The journal of physical chemistry B, 2016, vol. 120, no. 46, pp. 11942-11950, ISSN 1520-5207 Chart 1. | 1-20 |
| A | WO 2008/075016 A1 (CASTROL LIMITED) 26 June 2008, pp. 12, 13, table 3 | 1-20 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27.04.2021 | 11.05.2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/008074

| Patent Documents referred to in the Report | Publication Date | Patent Family | Publication Date |
|---|---|---|---|
| WO 2016/068336 A1 | 06.05.2016 | US 2017/0239173 A1<br>claims, examples, paragraphs [0005]-[0014]<br>EP 3213769 A1<br>EP 3395368 A1<br>EP 3395369 A1 | |
| JP 2015-501328 A | 15.01.2015 | US 2014/0356289 A1<br>claims, examples<br>US 2020/0214988 A1<br>GB 201119032 DO<br>WO 2013/064837 A1<br>EP 2773451 A1<br>AU 2012330894 A<br>CA 2853966 A<br>CN 104053497 A<br>KR 10-2014-0097303 A<br>IN 3680CHN2014 A<br>BR 112014010811 A | |
| WO 2008/075016 A1 | 26.06.2008 | US 2010/0093577 A1<br>EP 1970432 A1<br>EP 2126013 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020015688 A
- WO 2009066457 A
- JP 2020056457 A